# EUROPEAN PATENT SPECIFICATION

(11) **EP 2 032 093 B1**
(45) Date of publication and mention of the grant of the patent: **04.09.2013**
(21) Application number: 07795016.0
(22) Date of filing: 18.05.2007
(51) Int. Cl.: A61F 5/00

(54) **BIOERODIBLE INTRAGASTRIC IMPLANT**
BIOERODIERBARES INTRAGASTRISCHES IMPLANTAT
IMPLANT INTRAGASTRIQUE ET BIOÉRODABLE

(30) Priority: 18.05.2006 IL 17577806; 19.06.2006 IL 17639806; 13.07.2006 IL 17685606; 11.09.2006 US 519508; 19.04.2007 US 788574
(43) Date of publication of application: 11.03.2009
(73) Proprietor: Tulip Medical Ltd., 46722 Hertzelia (IL)
(72) Inventor: MARCO, Doron, 69626 Tel Aviv (IL); ECKHOUSE, Shimon, 31999 Haifa (IL)
(74) Representative: Price, Nigel John King
(86) International application number: PCT/US2007/011882
(87) International publication number: WO 2007/136735

(56) References cited:
- EP-A- 0 103 481
- WO-A-02/40081
- US-A- 5 129 915
- US-A1- 2003 109 935
- US-A1- 2004 186 502
- US-B1- 6 287 332

## Description

### FIELD

Described herein are bioerodible, biodegradable, or digestible self-deploying intragastric implants that may be swallowed. Once swallowed, the implants undergo self-expansion in the stomach and apply a suitable pressure against the stomach wall to provide a feeling of satiety to the individual. The internal components that provide gas for expansion of the implant may be deployed by mechanical mixing, fracture of a frangible component, manual manipulation, or the like. The implants then dissolve or are disassembled perhaps using gastric liquids and pass out of the stomach. Methods of using the devices, perhaps for an individual participating in a dietary control regimen, are described.

### BACKGROUND

Obesity is a major health problem in developed countries. In the United States, the complications of obesity affect nearly one in five individuals at an annual cost of approximately $40 billion. Except for rare pathological conditions, weight gain is often directly correlated to overeating.

One strategy for controlling the individual's food intake is via the use of intragastric volume-occupying devices. Such devices are placed in the stomach and occupy a portion of its interior. Properly placed and sized, the intragastric volumes provide the patient with a feeling of satiety after having eaten only a smaller amount of food. Typically, the individual's caloric intake is thus diminished due to the subjective feeling of fullness. There are a number of available volume-occupying devices. Many must be introduced using surgical or other complex gastric procedures.

Intragastric balloons have been in clinical use for several years. Their success in the treatment of certain individuals with morbid obesity is well accepted.

Published U.S. Patent Application No. 2004/0186502, U.S. Pat. No. 6,981,980, and published PCT application WO/2006/020929, to Sampson et al, each disclose inflatable, intragastric volume-occupying balloons including a valve that provides fluid communication into the balloon from outside the body. The '502 application further discloses a method for occupying some amount of stomach volume comprising the step of inserting the deflated balloon into the stomach through the esophagus, inflating the balloon by introducing an activating liquid through the self-sealing valve. Each document describes selection of polymers allowing gastric erosion of the balloon and causing its subsequent deflation.

Published PCT Application WO/2006/044640, to Baker et al, shows an implant used as a bariatric device situated along certain walls of the stomach to induce a feeling of satiation.

Published U.S. Patent Application 2004/0192582, to Burnett et al, shows a composition and a device that expands in the stomach after swallowing and provides a temporary, erodible volume and consequent diminution of gastric volume in the stomach.

U.S. Pat. Nos. 6,271,278 and 5,750,585, each to Park et al, show compositions of swellable, superabsorbant-hydrogel composites that may be used in gastric retention treatments for obesity.

U.S. Pat. No. 4,607,618, to Angelchik, discloses an intragastric device made up of semi-rigid skeleton members, collapsible to a shape, and having dimensions suitable for endoscopic insertion into the stomach through the esophagus.

U. S. Patent No. 5,129, 915, to Cantenys, relates to an intragastric balloon that is intended to be swallowed and that inflates automatically under the effect of temperature. The Cantenys patent lists three ways that an intragastric balloon might be inflated by a change in temperature. First, a composition of a solid acid and of a non-toxic carbonate or bicarbonate is temporarily kept from the fluid in the stomach by a coating of chocolate, cocoa paste, or cocoa butter. The chocolate coating is selected to melt at body temperature. Secondly, a citric acid and alkaline bicarbonate composition coated a coating of non-toxic vegetable or animal fat melting at body temperature may be used. When in the presence of water, the composition is said to produce the same result as does the earlier-discussed composition. Third, the solid acid and non-toxic carbonate or bicarbonate composition may be temporarily isolated from water by an isolation pouch of a low-strength synthetic material which is to break immediately upon swallowing. Breaking the isolation pouches causes the acid, carbonate or bicarbonate, and water to mix and to react, thereby inflating the balloon. The balloon itself is said to be made up of a modestly porous, but non-digestible material that allows slow deflation.

WO/2005/039458 shows a gastric constriction device that is to be mounted exterior to the stomach and cause feelings of satiation due to pressure on the vigil nerves of the stomach.

WO/2005/101983, to Dharmadhikari, shows an expandable composition that may be used as a gastric retention system, with or without the presence of ancillary drugs.

U.S. Pat. No. 5,783,212, to Fasihi et al, shows an expandable, erodible polymeric composition that may be used in drug delivery systems.

U.S. Pat. No. 6,733,512, to McGhan, describes an intragastric balloon having erodible patches that allow self-deflation of the balloon after a chosen period of residence in the stomach.

EP-A-0103481 discloses a gastric implant according to the pre-characterising portion of claim 1.

None of the cited documents discloses the bioerodible intragastric implant deliverable to the stomach by conventional oral administration that is described below.

### SUMMARY

Described herein are devices for and related methods for curbing appetite and for treating obesity. These treatments may be used in providing selective medical care and obesity therapy and are specifically suited to treatment of an individual patient while taking into account an individual's eating habits; differences in the individual's daytime and nighttime behavior; physiological and mental characteristics, body size, and age.

According to the invention, there is a provided a compacted, swallowable, self-expanding temporary gastric implant according to claim 1.

The devices include cost effective, biodegradable, self-inflating intragastric implants for curbing appetite and treating obesity, constructed from one or more discrete expandable members that are erodible in the stomach.

### BRIEF DESCRIPTION OF THE DRAWINGS

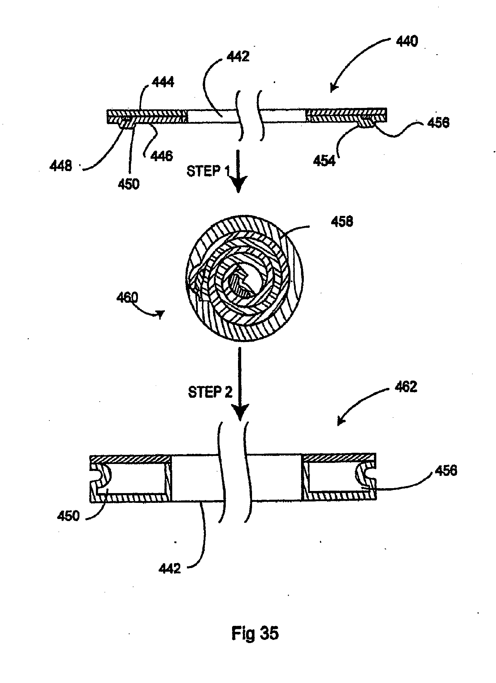

The invention is described with reference to the following Figures, in which Fig. 35 shows an embodiment of the claimed invention and the remaining Figures are useful examples for understanding the invention:
Figure 1 shows a perspective view of one variation of structural frame members suitable for use in an implant.
Figures 2A and 2B show, respectively, a perspective view and an end view of one variation of an implant comprising a structural frame member. Figures 2C and 2D show isolated sections of the Figure 2A and 2B implant.
Figures 3-11 show perspective views of several variations of structural frame members suitable for use in an implant.
Figures 12A and 12B show, respectively, a side view and an end cross-section view of one variation of a folded implant comprising a structural frame member.
Figure 13 shows a side view of one variation of a folded implant comprising a structural frame member, panels, and stabilizer members.
Figure 14 shows perspective views of a folded and expanded implant comprising a structural frame member and panels.
Figures 15A-15F show various panel structures.
Figure 16 shows a perspective view of a variation of an implant comprising a structural frame member and panels.
Figure 17 shows a top view of an unfolded implant comprising a structural frame member and panels.
Figure 18 shows a perspective view of an expanded implant comprising a structural frame member and panels.
Figure 19 shows a top view of an unfolded implant comprising a structural frame member and panels.
Figures 20A, 20B, and 20C show, respectively, a perspective view of a deployed implant, a top view, and a perspective view of a folded implant comprising a structural frame member and panels.
Figure 21 shows a top view of an unfolded circular implant comprising a structural frame member and a panel.
Figures 22A-22C show perspective views of several versions of unfolded implants each comprising a structural frame member and panels.
Figures 23 and 24 show, respectively, a top view and a perspective view of an unfolded coil-like implant comprising a structural frame member and panels.
Figures 25A-25C show, respectively, a perspective and two side views of substantively globelike implants.
Figures 26A, 26B, 26C, and 26D show, respectively, a top view before the joint is rotated, a top view after the joint is rotated, an end view, and a cross-section, side-view of a joint between two frame members.
Figures 27A and 27B show, respectively, a perspective view and a side cross-sectional view of two related self-inflating implants.
Figure 28 is a perspective view of a variation of an implant in an uninflated configuration.
Figure 29 is a top view of an implant variation shown in Figure 28 after inflation or expansion.
Figure 30 is a schematic representation of an appropriate placement of an implant within the stomach.
Figure 31 is a perspective view of another variation of an implant.
Figure 32 is a perspective view of a variation of an implant.
Figures 33A to 33C-1 provides perspective views of a several inflated variations of an implant.
Figure 33C-2 is a cross-section of the implant shown in Figure 33C-1.
Figures 33D to 33E provide perspective views of two inflated variations of an implant.
Figure 34A is a partial cross section of an expanded implant showing an expanded hoop portion and a container, at least partially contained in the hoop, containing a composition that produced an expanding gas.
Figure 34B is a perspective view of the container containing the expanding gas composition
Figure 35 shows the production of an implant that may be swallowed and the separation of the gas-producing composition components until appropriately mixed.
Figures 36-38 show side cross-sectional views of variations of valve members controllable in mixing components of the gas-producing composition.
Figure 39 shows a side cross-sectional view of a folded member for controlling the isolation of components of the gas-producing composition.
Figures 40A-40C show side cross-section views of frangible gas-producing components with fracturable outer shells, enclosing a single gas-producing chemical.
Figures 41A-41C show side cross-section views of frangible gas-producing components with various walls that are fracturable, enclosing two gas-producing chemicals.
Figure 42 shows a side cross-section view of a frangible gas-producing component with a single fracturable outer shell, enclosing a single gas-producing chemical, and positioned against and using a wall of the expandable implant as a barrier.
Figure 43 shows before-and-after, side cross-section views of a frangible gas-producing component with at least one interior, fracturable wall, enclosing two gas-producing chemicals, that causes consequential fracture of other component walls upon reaction of an initiator amount of one of the gas-producing chemicals.
Figure 44 shows before-and-after, side cross-section views of a frangible gas-producing component illustrating a fracture that opens a passageway between chambers enclosing two reactive gas-producing chemicals.
Figure 45 shows a close-up, side-view, cross-section of the fractured opening shown in Figure 44.
Figure 46 shows a side-view, cross-section of a pair of non-fracturable enclosures for the two reactive gas-producing chemicals separated by a fracturable joint of the type shown in Figures 44 and 45 and employing the outer implant wall as a barrier for the enclosures.
Figures 47A and 47B provide schematic representations of a bioerodible or digestible vehicle for *in situ* targeted delivery of effecters.
Figure 48 provides a schematic representation of a vehicle in which the entire enveloping layer is biodegradable.
Figure 49 is a schematic representation of a vehicle having an inner biodegradable layer enveloped by a second layer, such as a drug releasing or lubricating layer.
Figure 50 provides a schematic representation of a vehicle having a number of integrated enveloping layers.

### DESCRIPTION

Described herein are bioerodible intragastric implants that are useful in curbing appetite and that are constructed from one or more discrete expandable members. The implants may be swallowed and may be self-deployable. Also described are treatments for curbing an individual's appetite by using those devices.

The device curbs the appetite by temporarily expanding within the stomach to form a structure that contacts some portion of the stomach wall. When the implant is self-deploying, it expands in the stomach by being filled with a gas from a source that is swallowed with the device envelope or bladder. The described devices may be configured then to dissolve or to degrade after a selected period of time and be eliminated from the stomach.

The described self-deploying implant may be deployed in the stomach using, e.g., members comprising materials having "shape memory" characteristics or having significant elastic properties (e.g., elasticity or superelasticity of metals, alloys, or polymers) or other flexible constituent materials otherwise adapted to expand the device when placed in the stomach. The compositions are at least biocompatible and are desirably themselves bioerodible.

The device may comprise a single expandable member that expands upon introduction into the stomach or may comprise a plurality of discrete expandable members with attachments to other members or to a framework. Some variations of the device involve expansion of the member or members via use of an included amount of a carbon dioxide generating composition (CDGC).

Typically, the discrete expandable member of the type that is interconnected to another member is of one or more variations. In the first variation, the expandable member may be of a size and material, etc., that upon destruction of the connection to other members, it may be eliminated from the stomach without need for additional dissolution. In the other variation, the member is of a size and material in which further bioerosion is appropriate before the resultant member debris easily evacuates from the stomach.

As noted above, the various described members are desirably expandable in vivo upon contact with gastric juice. That expansion may take place over a predetermined and, perhaps extensive, period of time. The period of time during which the members erode to a size and form that passes from the stomach is similarly variable depending upon the result desired.

Expansion and dissolution times may each vary from about 0.25 hour to as much as 30 days. Obviously, the expansion times should be chosen to allow the stomach to activate the self-deploying feature of our devices before the stomach attempts to eliminate the device without deployment to the detriment of the stomach and the device.

Such predetermined time periods are to be selected by the medical specialist and reflect inter alia the size of the stomach, the age of the patient, the physical condition of the patient, food digestive parameters in the stomach and along the digestive tract, the medical condition of the stomach and its sphincters, the content of the gastric juice, and the patient's general physiological and mental condition.

The described devices are self-inflating or self-expanding and do not include external inflating valving for inflation of the implant from external sources after the implant has been deployed.

### Definitions

By the term "discrete expandable member," we mean that a specific member or component is itself expandable and may be temporarily interconnected to at least one other member, perhaps a structural element or perhaps as a portion of a structural element, by a bioerodible component, e.g., a tie.

By the term "bioerodible" we mean that the material is biodegradable, digestible, or erodible or otherwise dissolvable or degradable in the stomach to a form where the material is diminished in size by chemical, biological (e.g., enzymatic), physical dissolution, solubilization, etc. to allow elimination of the material from the stomach without substantial harm.

By the term "self-inflating" we refer to a spontaneous self-inflation feature. The implant need not include any inflating valves or the like, nor external inflating means.

By the term "pasta" we refer to mixtures of carbohydrates, e.g., flour and fluids, e.g., water, possibly with a predetermined measure of proteins, especially egg-related proteins.

By the term "gelatin" we refer to a protein product derived through partial hydrolysis of the collagen extracted from skin, bones, cartilage, ligaments, etc.

By the term "about" we refer to a tolerance of ±20% of the defined measure.

### Frame and Panel Structures

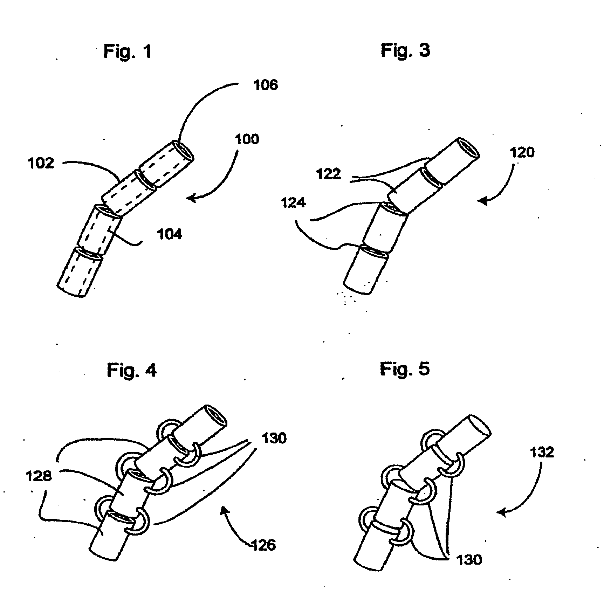

Figure 1 shows a flexible biodegradable structural member (100) comprising a number of hollow tubular members (102) having passageways (104) therethrough and joined by a thread, cord, or wire (106). The thread, cord, or wire (106) may have characteristics of high elasticity, superelasticity, or temperature shape memory allowing it to return to or form a desired shape upon being placed in the environment of the stomach. For instance, the structural member (100) may include a thread, cord, or wire (106) that includes shape memory characteristics allowing it to be compressed/twisted/folded or otherwise deformed into a small volume for introduction into the comparatively elevated temperature of the stomach and to re- form into a desired shape suitable for pressing onto the sidewalls of the stomach. The thread, cord, or wire (106) may comprise a bioerodible material or another material, as desired.

Expandable tubular members (102) may comprise a material that is expandable upon introduction into the stomach and is bioerodible to a form and size that may be eliminated from the stomach. Such materials are discussed elsewhere in a section specifically discussing those materials.

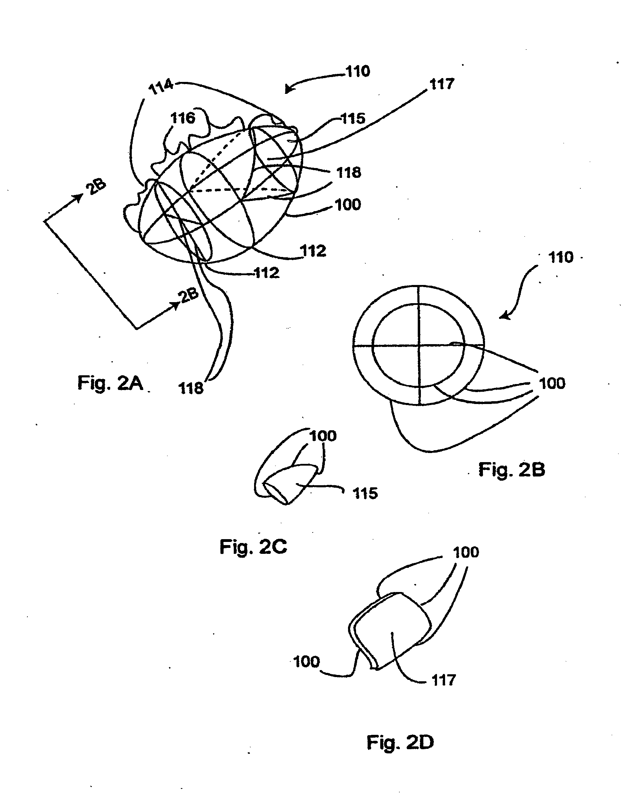

Figures 2A and 2B show an expanded framework (110) comprising a generally ovoid, football-shaped framework constructed from a number of structural members (100) or other similar structural members such as are discussed with relation to several of the following Figures.

Figure 2A shows a perspective view of the expanded framework (110) where the structural members (100) are joined at a number of locations (112) to form the desired shape. Other expanded shapes may also be apparent to the knowledgeable designer, e.g., stomach shaped, cup-shaped, etc. In any event, the end sections (114) comprise rounded triangular areas (115 also shown in isolation in Figure 2C) -- the edges being defined by the structural members (100).

Triangles are two-dimensional structural forms that are sturdy and support the form of the structure. In the inner two sections (116), the four-sided areas (117 also shown in isolation in Figure 2D) may be strengthened by exterior struts (118) and interior struts (120) if so desired or needed.

The expanded form (110) shown in Figures 2A and 2Bmay be used as shown or may be used as a framework for supporting panels or the like as will be discussed below.

Figures 3-12 show other variations of structural members of suitable for use in the same way as is the structural member (100) shown in Figures 2A and 2B.

Figure 3 shows a structural member (120) comprising a series of cylinders (122) secured to each other by ties comprising an elastomeric or rubbery material (124). As with the variation discussed with regard to Figures 2A and 2B, structural member (120) and the ties (124) may comprise a material that is expandable upon introduction into the stomach and is bioerodible to a form and size that may be eliminated from the stomach.

Figure 4 shows another structural member variation (126) comprising a series of erodible expandable tubular members (128) having a thread, cord, or wire (130) extending throughout.

The variation (126) also comprises several strengthening biodegradable wires (130) attached to the tubes (128) at the joints.

Figure 5 illustrates another variation of the structural member (132) having strengthening biodegradable wires (130) but without the thread, cord, or wire or securing material included in the variations shown in Figures 1, 3, and 4.

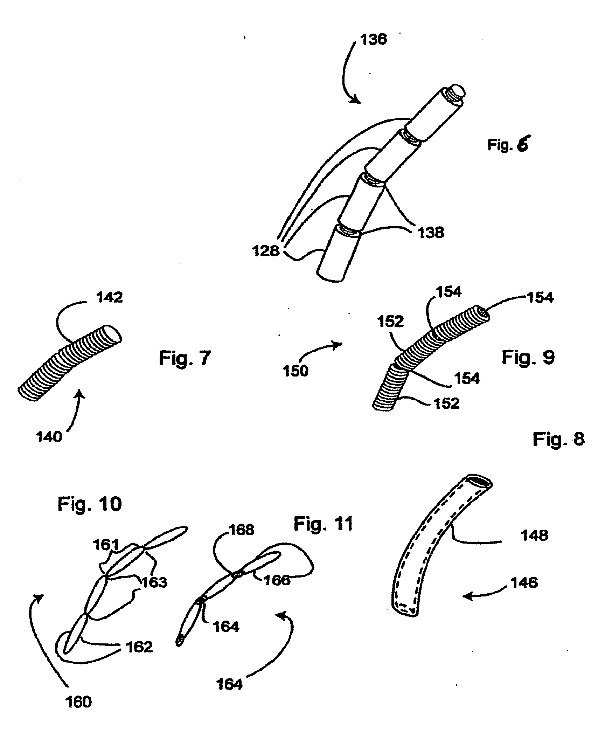

Figure 6 shows a variation of the structural member (136) having erodible expandable tubular members (128) but, unlike the other variations, has a coiled center member (138) linking the erodible expandable members (128). The coiled center member (138) may comprise a bioerodible material possibly having characteristics of high elasticity, super-elasticity, or temperature shape memory allowing it to return to or to form a desired shape upon being placed in the environment of the stomach.

Figure 7 illustrates a similar variation (140) wherein the structural framework comprises a spring-like member (142) comprising one or more biodegradable materials.

Figure 8 shows a variation (146) of the structural frame member comprising a hollow flexible biodegradable tube (148) full of gas. The tube (148) is filled with a gas upon introduction into the stomach using a self-contained gas source such as is discussed below.

Figure 9 shows a further variation of the framework (150) having multiple spring-like members (152) interconnected using ties (154) along its longitudinal axis. The ties (154) may comprise an elastomeric or rubbery material. As discussed elsewhere, the ties (154) may comprise a material that is expandable upon introduction into the stomach and that is bioerodible to a form and size that may be eliminated from the stomach.

Figure 10 shows a further variation of the framework (160) having sections (161) each comprising dual, substantially parallel elastic wires (162) separated by pinch-point joints (163). Again, the wires may comprise a bioerodible material. This framework variation (160) is able to be compressed or folded and to return to a desired shape upon release into the stomach.

Figure 11 shows still a further variation (164) of the framework in Figure 10 with sections, each section comprising dual, substantially parallel elastic wires (166) but having joints or flexible ties (168) joining the sections of wires. The ties (168) are of a material different than the wires (166).

As we discussed above, the structural frameworks shown above may be used in isolation as shown generally in Figures 2A and 2B. They may also be used as a support or structural frame for sheets or for inflatable envelopes or gastric balloons, as is discussed below. In any event, if the framework is used in isolation to provide pressure on the stomach wall, such a framework would be compressed, twisted, folded, or otherwise compacted or reduced to a form that may be swallowed by a patient.

The structural framework may also be characterized as ribs having ends, e.g., proximal and distal ends. Such ends may comprise sliding ends, in that they may be connected to other structural members in such a way that they slide with respect to that other member. For instance, the sliding ends may be forked or circular, with the other member situated within the open end of the fork or within the opening of the circular end. Such sliding ends may further comprise rotating hinges or a protruding element that cooperates with a slot or similar opening in the other element allowing a siding movement along the axis of that other member. Each of these ends may be used, dependent upon the design of the specific structure employed, to facilitate expansion or deployment of the implant structure in the stomach.

In some instances, the compacted form -- the form of the device before it is deployed in the stomach -- may be in a self-contained tension and require one or more components, denominated "shape stabilizers" to maintain that tension and to hold the compacted form in a swallowable form until reaching the stomach. The shape stabilizers may be made from materials that are bioerodible and desirably are of a form or shape that erode (and release) initially after being introduced into the stomach and therefore allow self-deployment.

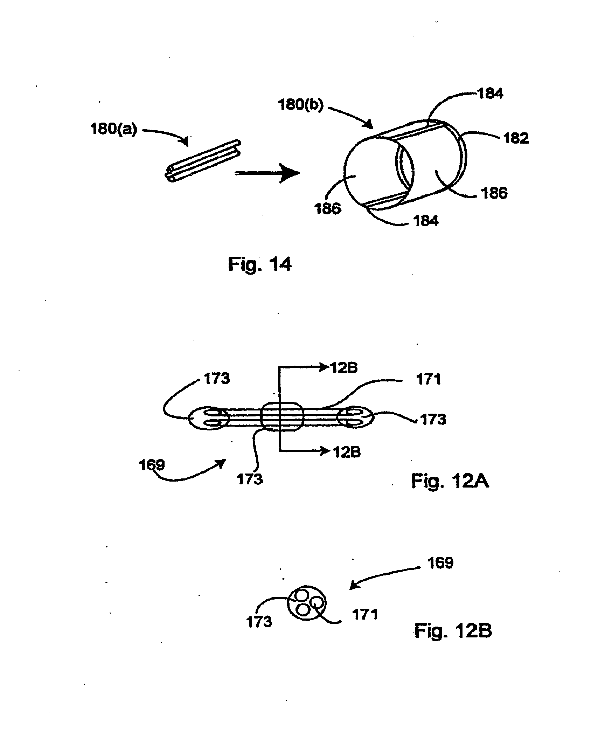

Figure 12A shows a generic representation of an implant assembly (169) that has been folded and compressed into a swallowable shape and size with a framework or set of structural members (171) and having a number of shape stabilizers (173) comprising a biodegradable material, e.g., gelatin or polyglycolide, that has been applied about the framework (171) to maintain it in a compressed condition until the stabilizer or stabilizers have eroded to allow self-implantation. Such shape stabilizers may be of a variety of forms, e.g., strings, ribbons, capsules, dried hydrogels (perhaps of gelatin or polyglycolide or the like) and other bioerodible forms.

Figure 12B shows a cross-section of the Figure 12A device showing, in particular, the structure of the stabilizing shape stabilizer (173) about the framework members (171).

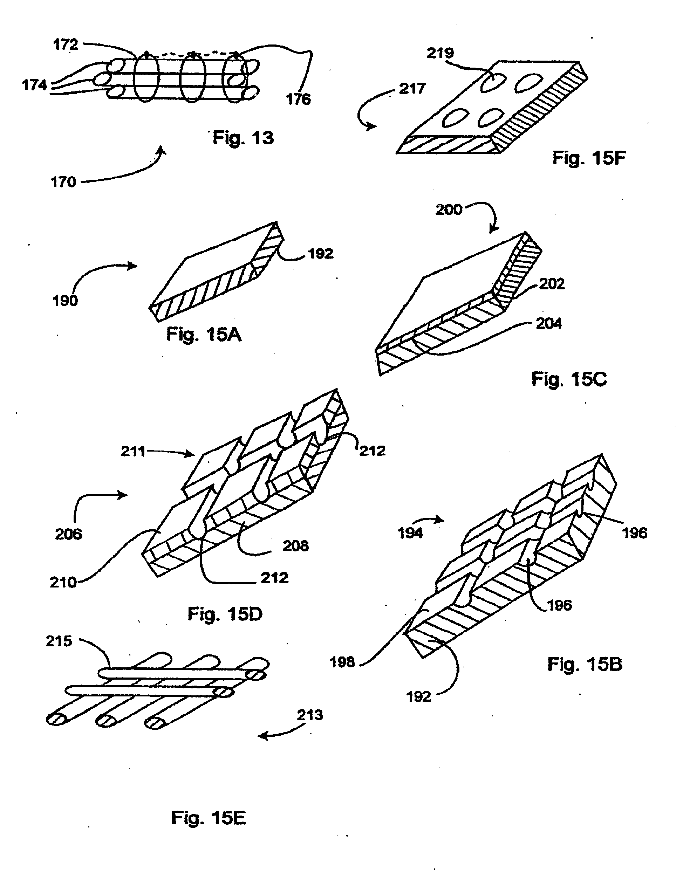

Figure 13 shows another folded device using shape stabilizers. Specifically, Figure 13 shows a folded framework (170) having a number of structural members (172) folded at ties (174) into a swallowable package and secured into that package by a bioerodible string or twine (176) that is looped about the package. The securing twine or string shape stabilizer (176) may optionally be tied using a "running" knot or "sack" knot. The securing twine or string (176) may be bioerodible to allow self-expansion of the folded framework (170).

Figure 14 shows a generic representation of the construction of an implant (180(a) folded and ready for placement in the stomach) and that implant (180(b)) after self-deployment in the stomach. The depiction of the implant after deployment (180(b)) shows several structural members -- circumferential structural members (182) and longitudinal structural members (184). Those members are connected to panels (186). Other variations of these generic structures are discussed elsewhere.

Figures 15A to 15F show representative cross-sections of materials that may be used in the panels or sheets mentioned elsewhere. The panels or sheets may be constructed in such a way that they are bioerodible in the stomach, bioerodible after passing through the stomach, or not bioerodible. The sheets or panels may be constructed in such a way that they have selectively erodible pathways that allow the structure to maintain its effective shape in the stomach and then to degenerate into smaller portions that may be further eroded either in the stomach or after passing out of the stomach.

Figure 15A shows a portion of a panel (190) comprised of a bioerodible material (192) that generally is to be substantially eroded in the stomach. Figure 15B shows a portion of a panel (194) comprised of a bioerodible material (192) that generally is to be substantially eroded in the stomach. A number of grooves (196) are included. The grooves (196) allow the panel (194) to be eroded in the stomach into smaller sections, e.g., section (198), that are non-structural in nature and may be eroded in the stomach. The grooves allow step-wise removal of the described implant from the stomach in a controlled manner.

Figure 15C shows another panel (200) made up of a layer (202) comprised of a comparatively more erodible material, perhaps of the same class of materials used in layer (192) in Figure 15A, and a layer (204) of a material that is in the class generally used as "enteric coatings." The enteric coating layer (204) is shown in the Figure as being on only one side; such a layer may be placed on both sides of the panel, though.

Figure 15D shows another representative panel (206) also having a layer (208) comprised of a comparatively more erodible material, again perhaps of the same class of materials used in layer (192) in Figure 15A, and an enteric material layer (210) having grooves (212) or other openings in the enteric coating into the more erodible material layer (208). The grooves (212) allow the panel (206) to be eroded in the stomach into smaller sections, e.g., section (211) that are non-structural in nature and may be eroded either in the stomach or after passage from the stomach. These grooves (212) also allow step-wise removal of the described implant from the stomach in a controlled manner. Again, the enteric coating layer (210) is shown in Figure 15D as being on only one side, such a layer may be placed on both sides of the panel (206) with or without the depicted grooves.

Figure 15E shows a representative panel (213) comprising filamentary components (215) stuck together to form an open weave material comprising one or more biodegradable materials. Such fabrics may be woven or nonwoven fabrics of filamentary materials as well.

Figure 15F shows another representative panel (217) comprising a sheet material having protrusions (219).

In general, the panels may comprise membranes or sheets, fabrics of assembled filaments (onlays, woven, or non-woven), meshes, screens, knits, etc. The panels may be stiff or very pliable. They may be layered constructs or neat materials. They may have physical properties allowing the expanded implant to provide pressure upon the stomach wall. They may be so highly flexible that they are easily twisted or compressed into small compact packages.

Materials suitable as "enteric coatings" are provided in a separate section herein.

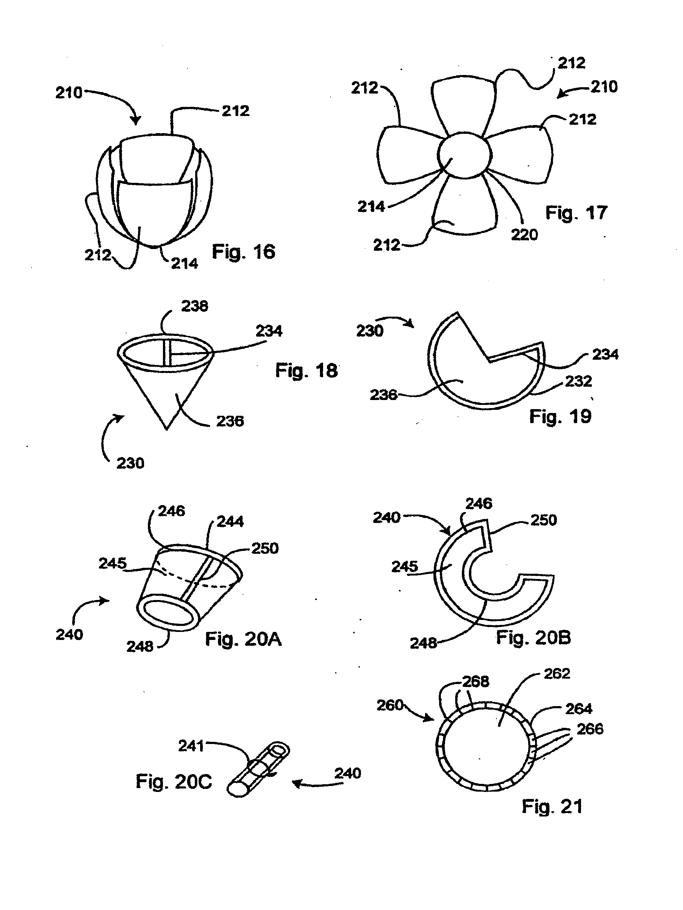

Figure 16 shows a form of an implant (210) that is cup-like in shape. The form comprises four panels (212) and a bottom (214, not visible in Figure 16). The depicted form may be considered as a schematic starting form in that it may be further compressed or folded. The panels (212) may be made of the compositions discussed above.

Figure 17 shows a flattened form of the implant (210) shown in Figure 16 with the side panels (212) and bottom (214). Structural members (218) around the edges of the panels (212) and a structural member (220) around the edge of the bottom panel (214) are also shown.

Figure 18 depicts a conical implant (230) as-deployed, with a circumferential support structure member (232) and a radial support member (234). The supported panel (236) is also shown.

Figure 19 also shows the implant (230) in the pre-formed shape having the circumferential support structure member (232), radial support member (234), and supported panel (236). The support members (232, 234) may be integrated into the panel (236) or linked to the panel (236) via tie members that may either comprise bioerodible materials or other biocompatible materials.

The conical deployed form shown in Figures 18 and 19 is an elected form. That is to say: the material making up the panel (236) may be "open" in the sense that it may be an "open weave" fiber material allowing fluid and solid flow through the field of the panel. The conical form allows deployment of the implant (230) in such a way that the circumferential structural member (232) rests against the stomach wall and imparts pressure against that wall providing a feeling of satiation. The "upstream" pressure of the liquids and solids inside the implant provides a continuing pressure against the circumferential structural member (232) until that member erodes sufficiently to pass from the stomach.

The radial structural member (234) provides a measure of directionality to the placement of the implant (230) in the instance where the connecting joint (238) provides some measure of rigidity to the unfolded structure of the implant (230). If radial member (234) is sufficiently long, e.g., longer than the diameter of the stomach and the connecting joint (238) is rigid (i.e., the joint sets the angle of the radial member (234) with respect to the circumferential member (232) as shown in Figure 18) or allows the radial member (234) only a small amount of rotation with respect to the circumferential member (232), the radial member (234) acts as a "director" and tends to align the circumferential member (232) across the stomach and to press the member (232) against the stomach wall.

As a design choice, the radial member (234) may be omitted so to allow the flow resistance of the panel (236) to align the circumferential member against the stomach wall, albeit with a slower alignment rate than with a device having the radial member.

Figures 20A, 20B, and 20C show a truncated conical implant (240) having a smaller opening (242) and a larger opening (244). The implant (240) also includes a panel (245), larger circumferential support member (246), smaller circumferential support member (248), and longitudinal support member (250). The truncated cone shape permits passage of stomach contents through the stomach without substantial interference and yet still utilizes that flow to align the implant in the stomach, in that the modest flow resistance tends to push the large end against the stomach wall.

Figure 20C shows a collapsed implant (240) of the type shown in Figures 20A and 20B with a shape stabilizer (241) ready for introduction into a patient.

Figure 21 shows a circular implant (260) having a panel (262) and an exterior circumferential support structure (264). The circumferential support structure (264) comprises, in this variation, a series of segments (266) joined by ties (268) as discussed above with respect to Figures 1 and 3-5. In this instance, the panel (262) may be perforated, with one or more openings through the panel. Such a panel includes the further advantage of slowing movement through the stomach in addition to the benefit of pressing upon the stomach wall.

Central to the use and configuration of our implant is the concept that the implant size and configuration be appropriate and sufficient to provide a pressure on the stomach wall to initiate and to continue that pressure prior to its shape degradation and passage from the stomach. Several suitable expanded implant shapes are discussed above with regard to Figures 2A, 2B, 14, 16, 18, and 20A.

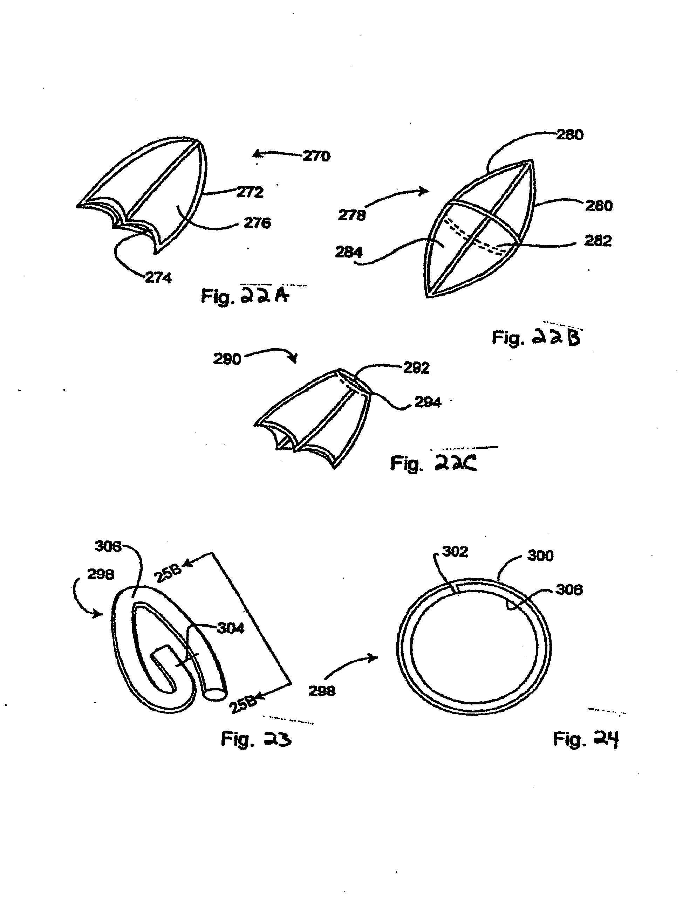

Other suitable expanded implant forms particularly useful in framework and panel constructions are shown in Figures 22A-25C. Still other suitable forms are discussed below.

Figure 22A shows an expanded implant form (270) having a generally half-football form comprising longitudinal structural members (272), partially circumferential structural members (274), and several panels (276). The partially circumferential structural members (274) generally provide pressure to the stomach wall.

Figure 22B shows an expanded implant form (278) having a football-like form comprising longitudinal structural members (280), a generally central circumferential structural member (282) that may be made up of several partially circumferential members, and several panels (284). In this variation, the circumferential structural members (282) may be configured to provide pressure to the stomach wall although the various panel (284) sizes and expanded stiffnesses may be chosen to form a structure that will provide pressure on the wall.

Figure 22C shows a truncated football expanded implant form (290) similar in overall form to the variation shown in Figure 22A with the general exception that the small end has an opening (292) potentially with a circumferential structural member (294).

Figures 23 and 24 show a spiral configuration (298) of our implant comprising a biased, structural member (300) of a spinal form that tends to open when in a relaxed condition. The configuration may include one or more smaller, circular, stiffening members (302). The structural member (300) may be considered to be a spring in compression, when deployed, and is held in the depicted, stressed form by halter filament (304). The implant is shown with a columnar panel (306) that is twisted into a spiral. Another structural member (not shown) may also be employed, e.g., on the inner circumference of the spiral column and attached to the circular members, to provide additional structure to the implant.

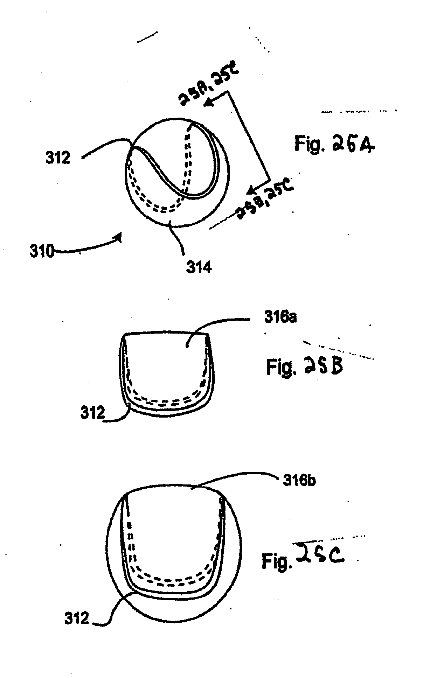

Figures 25A-25C show a variation of our implant (310) including a structural member that is springy (312) and that is easily twisted into smaller circular forms for introduction into the stomach. The implant (310) is shown with a generic panel (314), in perspective view, to allow overall understanding of the simplicity of the construction. Figure 25B shows the implant with a flexible sheet for the panel (316a). Figure 25C shows the implant with comparatively stiffer sheets for the panel (316b).

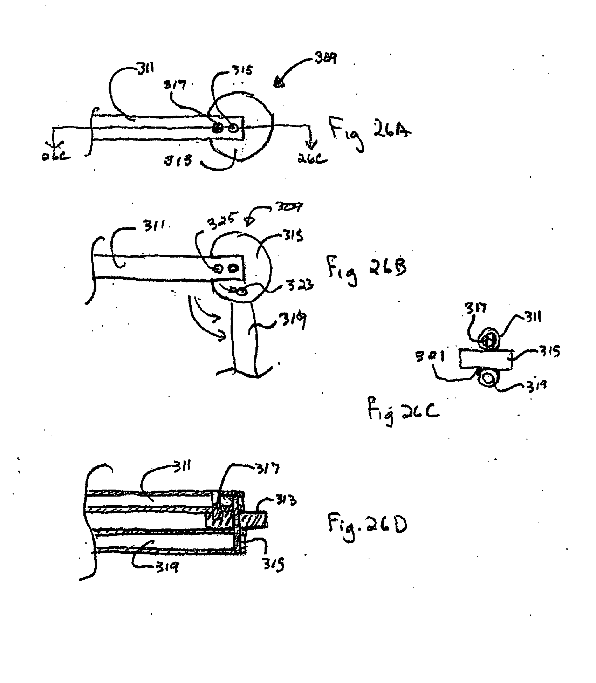

Figures 26A-26D show a connector that may be placed, e.g., between two structural members. The joint component of the connector rotates after the implant is introduced into the body to set the members in place.

Figure 26A shows a top view of our connector (309) and one of the structural members (311) to which it is attached. The structural member (311) and the joint (313) have a common axle (315) about which structural member (311) rotates after plug (317) erodes.

Figure 26B shows the connector (309) after the two attached frame members (311, 319) have rotated with respect to each other.

Figure 26C shows an end view of the connector in the form as seen in Figure 26A. The two attached frame members (311, 319) may be seen as may the plug (317) as it extends into the joint (313). A structural adhesive (321) attaching frame member (311) to the joint (315) may be seen. Frame member (319) may also be attached in the same way.

Figure 26D shows a cross section of the Figure 26A configuration with frame members (311, 319), the joint (313), and the erodible plug (317).

This joint is "loaded," locking the two frame members (311, 319) in the position shown in Figure 26A by twisting the frame members (311, 319) about the axle (315) passing through the rubbery joint (313) and inserting the erodible plug (317) through the opening (325) in the frame member (311) after it is aligned with the opening (323) in the joint (313). This twisting stresses the joint (313); eroding the plug (317) relieves that stress via the rotation.

Although the rubbery joint (313) may be made from a variety of materials, it should be structured of materials that erode more slowly than the plug (317). For instance, the joint may be an erodible material of, e.g., PLA-PGA, partially coated by, e.g., a rubbery material such as a silicone. The plug (317) may be of an erodible material, PLA-PGA.

The framework system discussed above is suitable for expanding, or to aid in supporting, variously the sheet structures specifically listed above as well as self-expanding envelope structures exemplified above.

### Self-Inflatable Structures Having Integral Gas Inflation Sources

Another variation of our implant comprises an efficient, self-inflating, or self-expanding implant that temporarily self-expands within the stomach of a patient using an integral gas source. Generally, when this variation of our implant is properly designed and sized, it will self-expand to a shape or to a diameter where the implant's perimeter is at least partially in contact with the interior surface of the stomach wall and thereby exerts at least a pressure on that portion of the stomach wall sufficient to trigger "fullness" responses from the stomach's nervous system. Functionally, the implant is sized to produce such a feeling in a particular patient variously before or after a patient has eaten.

After the implant has been in place in the stomach for a predetermined period of time, the implant will bioerode and pass through the stomach after being partially or completely digested. To accomplish this goal, the implant may be made from one or more bioerodible materials such as those listed herein, perhaps in combination with one or more enteric materials, again, such as those listed herein.

The expanded implant may comprise, although it need not necessarily be so, a structure having a comparatively larger size in two dimensions and a smaller size in the third direction.

When properly designed, this variation of the implant will provide a feeling of satiety, with the goals of not producing an uncomfortable feeling of fullness and without substantially changing conditions in the stomach, conditions such as temperature, digestive movements, pH, water activity (a_{w}), water availability in the stomach, and biological conditions such as the availability of digestive enzymes.

The implant self-expands due to the presence of an integral gas source. The gas source is "integral" in the sense that the components that produce the gas by chemical reaction, or biological process, or the like, are within the swallowed envelope or are in fluid communication with the envelope that forms the exterior of the expanded implant. Further, the term "integral" means that the implant's expanding gas is the product of a chemical or biological process based on gas-producing materials swallowed with the implant. The gas is neither added nor supplemented by a physical connection through a stomach opening. The integral gas source does not utilize gastric fluids as reactants in the gas source nor does the design of the implant permit the entry of gastric fluids into the interior of the un-inflated implant. External manipulation, e.g., via manual pressure or the like, may be used to initiate operation of the integral gas source in some variations, but such manipulation does not include use of any tool or means that is physically introduced into the stomach via the esophagus or via surgery. Such manipulation may, however, include the use of magnets or of applied radio-frequency energy or of physical manipulation of the swallowed implant via pressing with the hand or fingers to initiate, enhance, or continue the gas production process.

Although the gas source may, in a general sense, produce any gas that will inflate or expand the implant bladder or envelope, from a practical and safety point of view, a very good choice for such an expansion gas is carbon dioxide. The solid or liquid components suitable (and readily available) for producing carbon dioxide gas may be readily selected from materials that are themselves safe for human consumption. For instance, a suitable carbon dioxide source may comprise physically separated amounts of bicarbonate of soda and of a vinegar solution. These two materials are safe for human consumption, have no reaction products that are harmful, and are quite effective in producing carbon dioxide gas in amounts and at pressures that are useful in our implant. Only relatively small amounts of the reactants are needed to inflate the implant variation.

Chemical reactants involving simple acid-base reactions to produce carbon dioxide that are especially suitable for the gas source include: basic materials such as sodium bicarbonate (NaHCO₃), sodium carbonate (Na₂CO₃), calcium bicarbonate (Ca(HCO₃)₂), calcium carbonate (CaCO₃), and the like. Suitable acidic materials include various organic acids such as those found in vinegar (acetic acid), citrus juices (lime, lemon, orange, grapefruit juices containing citric acid), Vitamin C (ascorbic acid), tartaric acid, etc. Other organic acids, especially carboxylic acids are also useful. Carbonic acid is also operable but is, as a practical matter, too unstable at room temperatures to be a first choice for the gas source.

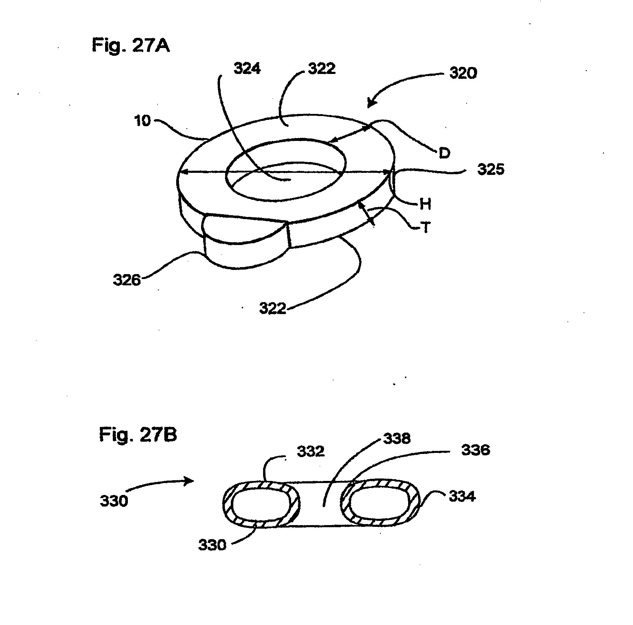

As to the structure of this variation of our implant, Figure 27A shows a perspective view of a flat-sided variation (320) comprising opposing, generally flat surfaces (322), an opening or passageway (324) through the implant to allow passage of stomach contents past the implant, and an outer periphery (325). The variation shown in Figure 27A also includes a stub or extension (326) that may include one or more components of the gas source.

Figure 27A also shows the conventions we use in describing the various dimensions of this variation of the implant (320). The dimension "H" is the diameter of the device. The dimension "T" is the thickness of the device and the dimension "D" is the difference between "H" and the diameter of the inner opening (324).

The depiction of the implant (320) shown in Figure 27A depicts an outer periphery (325) having a relatively right-angled shoulder or edge. Figure 27B shows a cross-section of another implant variation (330) in which the expanded form has opposing surfaces (332), an outer generally rounded periphery (334), and a rounded inner surface (336), surrounding a central opening or passageway (338). This variation (330) may be referred to as having a toroidal or donut shape.

In general, these variations of our implant, when expanded, comprise a configuration that has a generally flat shape in which D<H and T<H, the external periphery (325, 334) of which is selected to press against the sidewalls of the stomach. Typically, the "H" dimension of the expanded or inflated envelope is functionally in the range of about 75% to about 110% of the inner latitudinal diameter of the stomach, typically less than about 15 cm. The pre-inflated envelope or bladder (but not yet rolled or otherwise folded into the swallowable form) may have "T" and "D" dimensions of less, even substantially less, than about 2 cm.

The functional and physical sizes mentioned here with respect to the self-inflating implant variations of the device are, of course, based upon the implant's relationship with the size of the stomach. These sizes are also suitable for the frame-based variations discussed above.

The dimensions of such a partially or fully expanded implant may vary, e.g., the "D" dimension may be between about 0.2 and about 3.0 cm, perhaps between about 1.2 and about 1.8 cm, and perhaps between about 0.75 and about 1.5 cm; the "H" dimension typically will be between about 8 and about 18 cm, perhaps between about 11 and about 14 cm.

Depending upon the materials of construction chosen and their various thicknesses, pre-inflation collapsed implant volumes may be in the range of 0.02 cm³ to about 5.0 cm³ and perhaps in the range of about 1.0 cm³ to about 2.0 cm³. These dimensions allow the implant, when properly collapsed, to be swallowed by a patient.

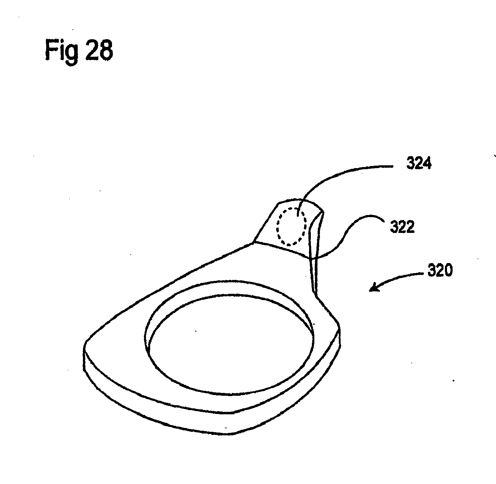

Figure 28 provides a perspective view of the variation of our implant (320) shown in Figure 27A, differing in that the device is in a collapsed form and has not yet self-inflated. Again, the depicted implant will yet be collapsed further so to be swallowable. Also visible in Figure 28 is an optional fold line (322) and a component (324) of the gas generating composition discussed above. The fold line (322) may be used, if desired, to provide an added restriction preventing contact between the components of the gas generator prior to the implant's (320) unfolding in the stomach. The use of the fold line in this and certain other variations will be explained in more detail below.

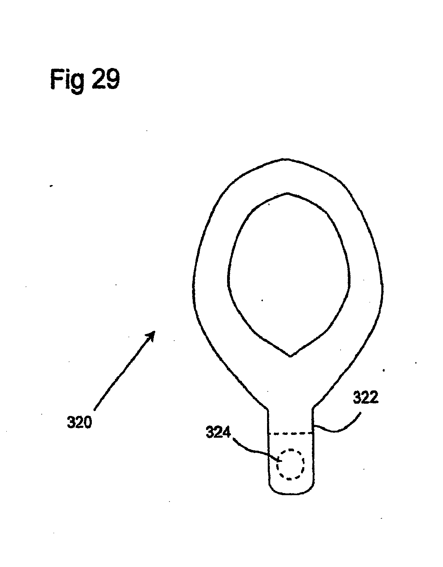

Figure 29 shows a top view of the expanded implant (320) otherwise shown in Figures 27A and 28. Fold line (322) and a component (324) of the gas generating composition as discussed above are also shown.

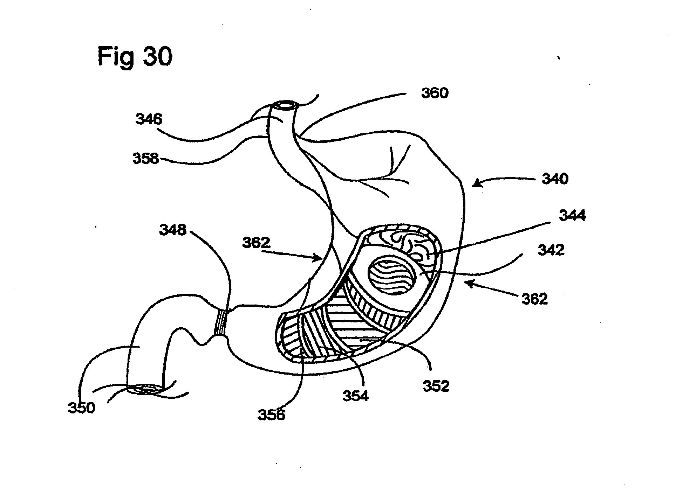

Figure 30 shows a partial sectional view of a stomach (340) with our self-deployed implant (342) in place against the stomach wall (344) showing the folds of the mucous membrane. Additional anatomical features of the stomach shown are the entry to the stomach (340), the esophagus (346), and the exit of the stomach (340), the pylorus (348). The pylorus (348) leads to the duodenum (350). The various layers of stomach muscle (352, 354, 356) are also shown.

The nerves in the stomach (340) are the terminal branches of the right vagus nerve (358) and the left vagus nerve (360). The right vagus nerve (358) is distributed on the back of the stomach and consequently those distributed nerve branches are not seen in Figure 30. The left vagus nerve (360) branches on the front of the stomach (340) and those branches are depicted in Figure 30 on the front surface of the stomach (340). A great number of branches from the celiac plexus branch of the sympathetic nerve are also distributed to the stomach. Nerve plexuses are found in the submucous coat in the stomach wall (344) and between the various muscle layers (352, 354, 356). From these plexuses, nerve fibrils are distributed to the muscular tissue (352, 354, 356) and the mucous membrane (344). The interior lateral diameter "ILD" (362) of the stomach (340) is also seen.

It is the pressure on these highly branched nerves that the implant (342) is to provide in operating to provide a feeling of satiation.

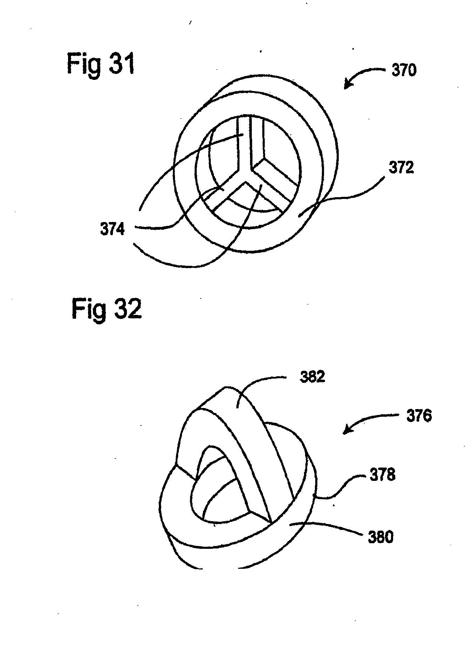

Figure 31 shows a perspective view of another variation of our self-expanding implant (370) in its expanded form. The toroidal outer shape (372) is given accentuated diametric stiffness by the three radial ribs (374).

Figure 32 shows a perspective view of another variation of the self-expanding implant (376) having a flat ring or toroidal region (378), the periphery (380) of which is configured to contact the stomach wall. A half-loop (382) helps to maintain the regularity of the toroidal region (378) and is available to provide pressure against the stomach wall if the alignment of the toroidal region (378) of the implant (376) is not directly across the stomach.

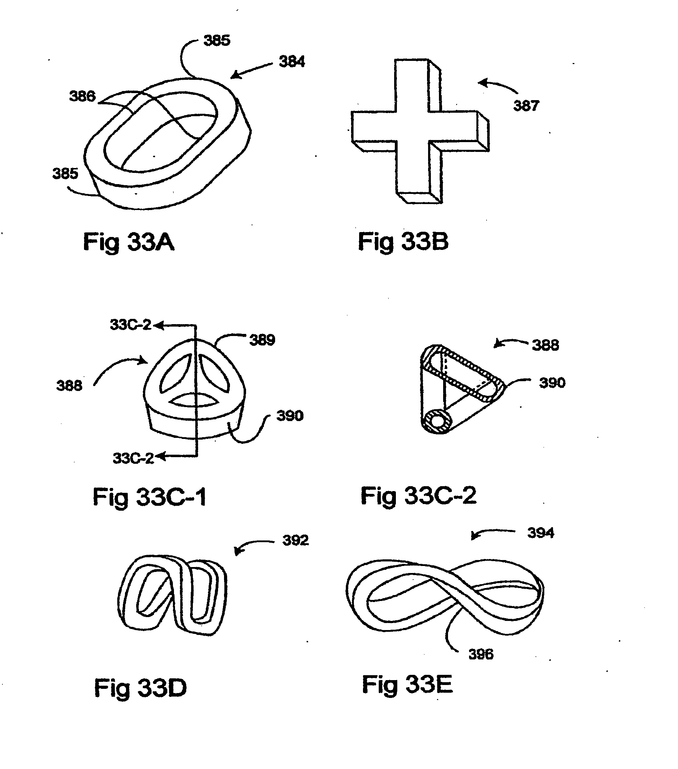

Figures 33A-33E show a number of expandable envelope shapes that may be inflated with a modest amount of gas and by extension, using small amounts of reactants. One significant advantage of the toroidal or donut-shaped inflatable envelope discussed above is that the package to be swallowed may be made quite small and yet the exterior dimensions are comparatively extensive. That shape is able to provide a stable structure able to provide significant and widespread pressure on the stomach wall for producing the "fullness" sensation. The shapes found in Figures 31 and 32 and in Figures 33A-33E also provide such advantages.

Figure 33A shows a perspective view of an expanded implant (384) that has semi-circular ends (385) separated by two relatively straight sections (386). The rounded ends (385) provide surfaces that are broad and cover large sections of the stomach wall allowing good predictability that some portion of the stomach nervous system will be under pressure. The straight sections (386) are, in effect, structural beams that press the ends (385) against the stomach wall.

Figure 33B shows a perspective view of an expanded implant (387) having the shape of a multiple-armed cross. The cross arms extends to press various generally opposing areas of the stomach wall. It is a sturdy structure.

Figure 33C-1 shows a perspective view of an expanded implant (387) having the shape of skeletal 3-pyramid. Figure 33C-2 shows a cross-section of the Figure 33C-1 implant. This shape is a determinate form and has rounded corners (390) for contacting the stomach wall.

Because the triangle-based pyramid shape includes a determinate structure, the shape provides a variety of sites for pressing into the stomach wall.

Figure 33D shows a perspective view of an expanded implant (392) having the shape of a bifolded disc.

Figure 33E shows a perspective view of an expanded implant (394) having the shape of a Moebius strip. The twist (396) in the form provides a measure of stiffness to the overall structure.

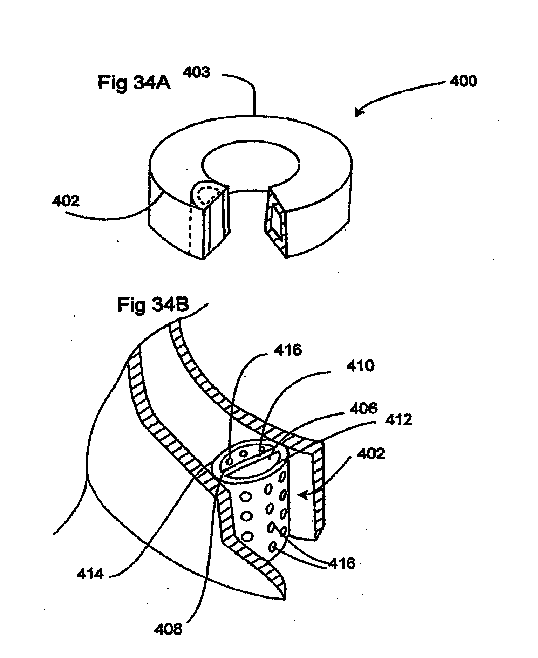

Figure 34A provides a partial cutaway view of a hoop- or ring-shaped variation (400) of our expandable implant and further shows an alternative placement of the gas generator (402) within the hoop. This gas generator configuration is shown in Figure 34A is further shown in the cutaway of Figure 34B. The container or canister (402) includes two volumes (406, 408), each of the volumes containing one of the reactants, e.g., base or acid. The two reactant-containing volumes (406, 408) are separated by the wall (410). Each of the opposing, rounded walls (412, 414) includes a number of openings (416) that either allows a liquid reactant in or out. In any case, a liquid reactant present in volume (408) leaves that volume through the openings and must travel completely around the interior of torus (403 in Figure 34A) to approach the other side of container (402) and to pass through the openings in the opposite volume (406) and initiate the reaction to produce the inflating gas. This arrangement permits ease of gas producer canister (404) installation and yet separates the reactants by a maximum path.

Figure 35 shows a side cross-section of a self-expanding implant in three stages to demonstrate a variation for placing the two components of the gas-producing composition in isolation until appropriate for reaction and resultant gas production.

Shown as the starting construction of the Figure 35 procedure is an uninflated implant (440) having a central opening (442) with an upper flat surface (444) and a lower flat surface (446). A first component (448) is located in a first volume (450) and a second component (454) is located in the remote second volume (456).

In "Step 1," the uninflated implant (440) with the included first component (448) and the second component (454) is then rolled tightly while maintaining the isolation of the two components. The rolled implant (440) is then coated with a biodegradable material, e.g., a gelatin layer (458) to form a coated swallowable implant (460). Although the liquid may be expected to exhibit some tendency to migrate between the layers forming the upper (444) and lower surfaces through capillary action to the reactive other component, it is our experience that such occurrence is rare. The tighter the roll, the less tendency has the liquid to migrate. Although not wishing to be bound by this theory, we conjecture that any reaction that takes place through liquid seepage raises the system pressure within the rolled device and pushes the liquid back into its volume.

In "Step 2," the coated implant (460) is swallowed and the bioerodible covering (458) is dispersed allowing the tightly rolled implant to unroll and to allow the first component found in the first volume to move to and to react with the second component found in second volume (456) to form a gas, inflating the device and form an inflated implant (462).

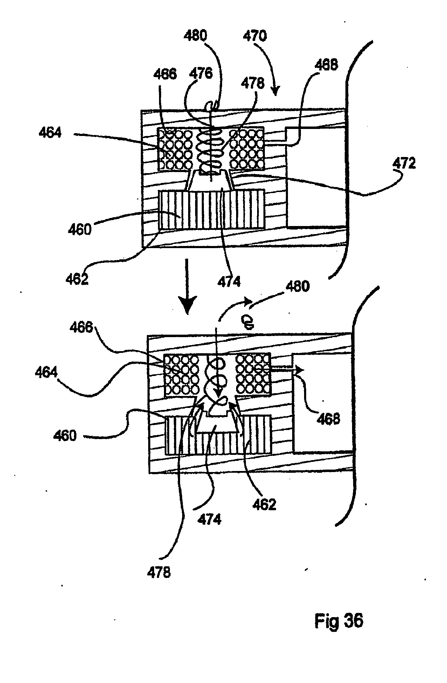

Figure 36 shows another schematic device for self-inflating the implant. As is the case above, at least one of the components is liquid. In this variation, a first, liquid component (460) is located in a first volume (462) potentially having a closable opening or openings only into a second volume (466) containing and a second liquid or solid component (464). The second volume (466) includes a passageway (468) into the inflatable bladder or envelope forming the implant. The first component (460) and the second component (464) together comprise the gas-producing composition.

The passageway (472) between the first volume (462) and the second volume (466) is temporarily closed using a plug (474) that is held in a closed position using a thread (476). Spring (478) pushes upon plug (474) and thereby biases the plug (474) to open. Thread (476) pulls against the spring. If thread were not there, as is the case in the lower panel of Figure 36, the spring (478) compression would open the plug (474).

Exterior to the implant is a digestible knot (480) or the like that, once digested or eroded in the stomach as shown in the lower panel of Figure 36, allows the thread (478) to slip through the upper wall and, in turn, allows the spring (474) to push the plug (474) into the first volume (462) and displace some of the first liquid component (460) into the second component (464) in second volume (466) producing inflating gas that escapes through opening (468) to inflate the implant (470). The spring (474) may be formed of a biodegradable polymer such as polyglycolic acid.

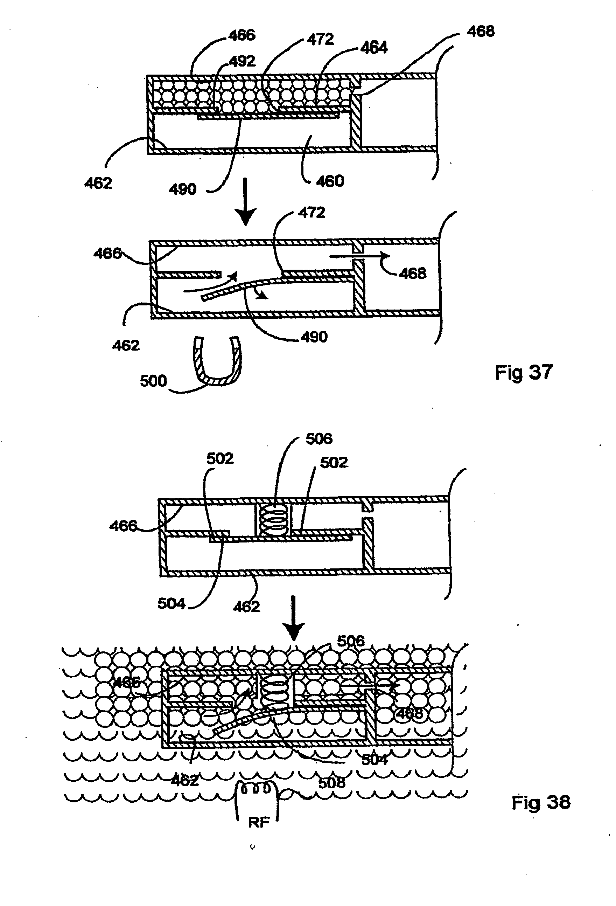

Figure 37 shows a generally similar arrangement to that of Figure 36, but with a different sealing or valving arrangement. In this variation, the passageway (472) between the first volume (462) and the second volume (466) is temporarily closed using a flexible, polymeric, magnetic flapper (490) optionally having a magnetic seat (492) surrounding opening (472).

The flapper (490) may comprise a polymeric, flexible material including, for instance, ferromagnetic particles. The ferromagnetic particle loading should be sufficient to allow a magnet (500) move the flapper (490) away from opening (472) as shown in the lower panel of Figure 37 and to allow fluid (460) from first volume (462) into second volume (466) for reaction to gas ultimately flowing through opening (468) to inflate the implant. In practical operation, a magnet could be applied to a patient's stomach area to open flapper (490) and initiate gas production.

Figure 38 shows still a similar arrangement to that shown in Figure 37 but instead utilizing a thermoplastic sealant (502) to hold flapper (504) in place against a spring (506) in compression until released by application of radiofrequency energy (508) to melt the thermoplastic sealant (502) and to allow fluid (460) from first volume (462) into second volume (466) for reaction to gas ultimately flowing through opening (468) to inflate the implant. Desirably such a thermoplastic sealant (502) would have a fairly low softening point and be loaded with a material such as ferromagnetic or ferrimagnetic particles that tend to concentrate the RF energy into the sealant for softening.

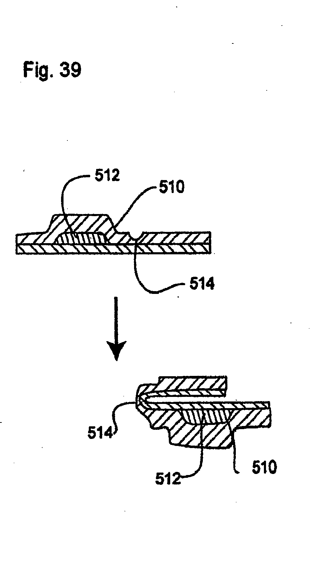

Figure 39 simply shows a volume (510) that contains one of the components (512) of the gas-producing composition and the presence of a fold line (514) to concentrate sealing forces at that line (514) when folded (as shown in the lower panel of Figure 39) and to maintain the isolation of the component (512) within volume (510).

The folded construct may simply be folded, as is, into a roll such as found in Step 2 of Figure 35 to help maintain isolation of reactants until needed for gas expansion.

Another set of structures suitable for maintaining separation between the components of the gas-producing composition are those we characterize as "frangible" structures. By "frangible" structures, we mean those structures that maintain separation between the two components until some exterior movement or pressure causes a physical fracture of at least some portion of the structure permitting contact between and consequent chemical reaction between the components of the gas-producing composition. As is the case with each of the gas-producing compositions discussed above, either or both of the components may be in a liquid, solid, semisolid, or gel form.

The impetus for physically fracturing some portion of the frangible structure may be any intracorporal movement or pressure, natural or applied. Pressures such as those caused as a result of peristaltic motion or the act of swallowing or physical pressure applied from outside the body (e.g., due to a health professional pressing on the stomach) may be used to fracture the fracturable component of the frangible structure. Movements such as that involved with unfolding the balloon after dissolution of the outer capsule, peristaltic motion, or physical manipulation of the frangible structure may be used to fracture the fracturable component of the frangible structure.

The region of fracture may be widespread, e.g., destruction of a structural wall in the frangible structure, or narrow, e.g., the opening of a small passageway or hole allowing contact between the gas-producing chemicals. The relative size of the region of fracture may be between these extremes.

A wide variety of materials are suitable for use in the fracturable component of the frangible structure. Waxes such as petroleum waxes, bees wax, polyethylene waxes, are suitable. Natural materials (or those derived from natural materials) such as hard gelatins, rice and wheat noodle materials, starch-based compositions perhaps comprising potato-starch with or without synthetic and natural polymers, may be used as some portion of the fracturable components. The fracturable component may comprise regions of non-fracturable materials (perhaps dissolvable in stomach fluids such as polyglycolic acid membranes) edged by fracturable materials.

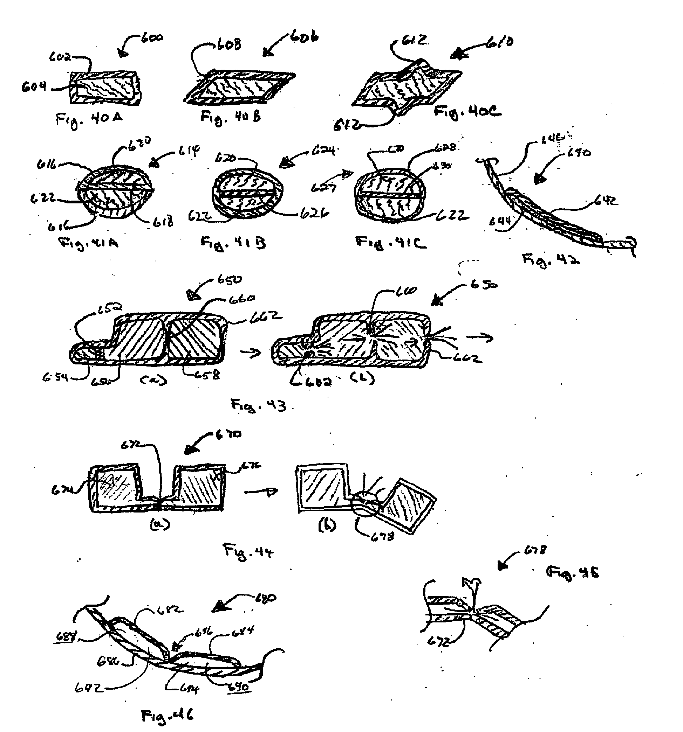

Figures 40A-40C show side-view cross-sections of three frangible structures having outer fracturable coverings. Figure 40A shows a generally square-cornered structure (600) having an outer fracturable cover (602) and containing one or the other of the gas-producing chemicals. Figure 40B shows a structure (606) having a parallelogram cross-section that, because of its non-right-angle corners, will fracture under pressure more easily than will the structure shown in Figure 40A. Figure 40C shows a structure (610) with regions (612) that provide ease of stress-fracture. The hillocks (612) need not have the specific shape shown there to achieve enhanced fracturably. Other shapes have stress-concentrating abilities.

Figures 41A-41C show structures in which one or more fracturable surfaces are used to maintain a separation between the gas-producing components. Figure 41 A shows a frangible structure (614) having two fracturable surfaces or walls (616) and a central wall (618), all combining to enclose a first gas-producing solid or fluid (620) and a second gas-producing solid or fluid (622). The central wall (618) may be fracturable or not. Figure 41B shows a frangible structure (624) in which a fracturable central wall (626) is used to separate a first gas-producing solid or fluid (620) and a second gas-producing solid or fluid (622). Figure 41C shows a frangible structure (627) having an outer surface or wall (628) and central wall (630) used to separate a first gas-producing solid or fluid (620) and a second gas-producing solid or fluid (622).

The structures shown in Figures 40A-40C and 41A-41C might be introduced into the expandable bladder, balloon, or envelope but not necessarily affixed to the interior of the envelope or balloon.

Figure 42 shows a frangible structure (640) in which a fracturable wall (642) contains one of the first or second gas-producing solid or fluid (644) in conjunction with a portion of the expandable envelope (646). Two (or more) of these fracturable walls may be used in a single expandable envelope to enclose selected amounts of the first or second gas-producing solid or fluids. The multiple structures need not be all of the same size.

Figure 43 shows before-and-after, cross-section, side-views of a frangible structure (650) having at least one interior fracturable wall (652). The overall structure of the device (650) includes three containment volumes or regions (654, 656, 658). The small volume (654) is isolated from the adjacent chamber (656) by fracturable wall (654) and contains either of an acid or a base. The adjacent or middle chamber (656) contains acid (if the small chamber (654) contains base) or a base (if the small chamber (654) contains an acid). The final chamber (658) contains an acid or base as is found in the small chamber (654).

This structure helps to enhance the mixing of the reactants. When the fracturable wall (652) breaks, as shown in part (b) of Figure 43, the small amount of reactant in volume (654) reacts with the complementary reactant in chamber (656) to form a gas and that expansion pushes the reactant in middle chamber (656) through a thin wall (660) between chamber (656) and chamber (658)) and into the end chamber (658) to cause reaction of the reactant in chamber (656) and that in chamber (658) and to form the larger bulk of gas. The expansion of the gas will force through thinned end wall (662) into the outer envelope for expansion of that envelope.

One or more of thin walls (660, 662) may be fracturable regions, if so desired.

Figure 44 shows before-and-after, cross-section, side-views of a frangible structure (670) having at least one interior fracturable joint (672) separating two volumes (674, 676) containing the two gas-producing chemicals. Bending the fracturable joint (672), as seen in portion (b) of Figure 44, both opens the passageways to the two volumes (674, 676) and opens the joint to the interior of the expandable envelope. Figure 45 shows a close-up side view of the fracturable joint (672) found in the device of Figure 44. These types of frangible seals have been used extensively. See, for instance, U.S. Patent No. 4,790,429, to Fukushima.

Finally, Figure 46 shows a frangible structure (680) in which a pair of walls (682, 684), adherent to the envelope wall (686), each define a volume (688, 690) containing one of the first or second gas-producing solid or fluid (692, 694). The walls are joined at a fracturable joint (696) of the type described in conjunction with Figures 44 and 45. Fracture of the fracturable joint (696) allows contact of the reactant gas-producing chemicals and expansion of the envelope.

### Methods of Use

The described gastric implant is used in the following fashion: the swallowable device is (a) administered orally to the patient in a collapsed configuration, (b) transported to the stomach, (c) expanded in the stomach to an approximate predetermined size providing pressure on the stomach wall, perhaps by expansion of an expandable member or upon erosion of a shape stabilizer, (d) disassembled after a predetermined period of time by bioerosion and (e) evacuated from the stomach.

The described devices may be used to curb appetite. This treatment comprises the step of periodic oral administration of biodegradable self-inflating intragastric implant constructed from one or more discrete expandable members. The intervals between each administration are determined by a medical professional after taking into account the physiological and mental characteristics of the patient. This treatment is especially useful wherein the deflated implant is swallowed before meals such that the food intake during the meal and afterwards is decreased.

### NON-GASTRIC IMPLANTS

In addition to the gastric uses discussed above, the biodegradable self-inflating or self-opening implant structures exemplified above may also be introduced into the patient through a natural or created body opening, via a body cavity, to a predetermined remote location. The implant for these non-gastric uses may utilize any of the structures discussed above.

These implants may be introduced into natural or created body openings such as the mouth, nose, ear, urethra, vagina, rectum, apertures in blood vessels provided for blood transfusion, apertures made by a cannula, syringe, needle, trocar, or any combination thereof.

In addition to the stomach, these implants may be passed through various of the respiratory tracts, urological, tracts, ear & nose tracts, GI tracts, vasculature, and body cavities made accessible by endoscopic or laparoscopic procedures.

As is the case with the gastric implants, these implants may be disassembled or biodegraded in a series of following or simultaneous events. The implant may comprise more than one type of biodegradable ingredient. For example, the components may be constructed of segments or layers or coatings of materials that biodegrade at different rates.

Although the materials and structure may be chosen so that the implants perform their disclosed function with any moisture present at the implant site, e.g., a urethral implant may be eroded with urine, fluids such as saline, water, with or without added adjuvants, e.g., medicines, diagnostics, etc., may be introduced at the implant site to cause the noted erosion of the implant.

### PLACEMENT OF EFFECTERS

Finally, the implant structures described herein may be used as vehicles for *in situ* delivery of effecters and implants to remote sites in the body. These structures, particularly in the form of digestible or biocompatible capsules accommodating expandable balloons or the like, are suitable for traverse to the GI tract or to other cavities of the body. Proper design of these specific implants may be necessary to so place the implant without premature expansion or inflation, prior to arrival at the target, e.g., in the upper GI tract or other targeted portions of a body cavity.

These erodible implants comprise or are adapted to provide digestible or bioerodible vehicles for *in situ* targeted delivery of effecters and are implanted by passage through a natural or created body opening, through a body cavity, to a predetermined remote location. The biocompatible vehicle at least partially envelops at least one effecter.

The vehicle may be at least partially biodegradable or biodegradable in the conditions found at the targeted remote location or created by the user at the target site.

Some or all of the enveloping capsule is biodegradable is biodegradable, collapsible, dismantlable, disintegrable, decomposable, or the like. A biodegradable portion of the enveloping capsule may be a segmented covering, a multi-layered coating, or any combination thereof.

The erodible implant may be used to deliver effecters such as one or more inflatable balloons (including gastric balloons), solids comprising slow-release drugs, diagnostic tools such as video cameras or temperature measuring devices or biopsy devices, remotely operable surgical tools, or other medically utilizable effecters adapted in terms of size and shape to be at least partially encapsulated within the vehicle and be subsequently implanted in a remote body location.

The implants may be introduced through body openings such as the mouth, nose, ear, urethra, vagina, rectum, apertures in blood vessels provided for blood infusion, apertures made by cannulae, syringes, needles, trocars, or the like. The described implants may then pass through such lumen as the GI tract, respiratory tract, urological tract, ear & nose tracts, body cavities made accessible by endoscopic or laparoscopic procedures, etc. The targeted locations include gastroenterological organs, including the stomach.

In the instance where the cover is an enveloping capsule, that erodible cover may comprise one or more of the enteric compositions listed above. Additionally, the cover may comprise additives, such as biocides, medicaments (including sustained released drugs), plasticizers, radio-opacifiers, oils, fillers, vitamins, hormones, factors, extracts (including plant extracts), co-factors, and mixtures thereof.

In the event that an oil is included as an additive, suitable oils include esters, such as stearyl glycyrhetinate, tocopheryl acetate, panthenol, phosphatidylcholine, glyceryl stearate, caproic/capric triglycerides, cetyl octanolate, isopropyl myristate, ceteatyl octanoate, butylene glycol dicaptylate/dicaprate, hydrogenated castor oil, monoglycerides, diglycerides, triglycerides, and the like; and vegetable materials, such as beeswax, carnauba wax, olive oil, jojoba oil, hybrid sunflower oil, mineral oil, squalene, squalane, monoglycerides, diglycerides, triglycerides, middle chain glycerides, myglyol, cremophor, hydrogenated castor oil, corn oil, soybean oil, sesame oil, cottonseed oil and fat-soluble vitamins.

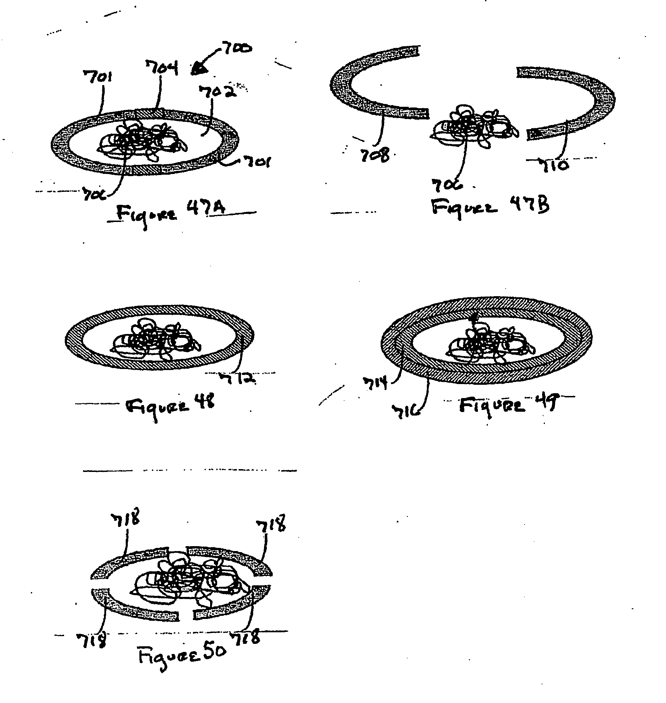

Figures 47A to 50 provide schematic illustrations of bioerodible vehicles for *in situ* targeted delivery of effecters.

Figure 47A shows a shape memory NiTi-based inflatable member (700) in its compact or pre-inflated configuration. The illustrated device is a gastric balloon that may be implanted from a body opening, pass via a body cavity, to a predetermined remote location. The biocompatible and bioerodible vehicle (700) envelops in its inner volume (702) one or more effecters (706) encapsulated within. The covering comprises two or more portions or sections (701) comprising non-biodegradable matter, e.g., PMMA, or a slowly degradable material, such as PLA. Those portions (701) may be affixed to each other by melting, gluing or by other mechanical or chemical interconnection. They may be integrated with each other. They may be made to adhere to each other using a biodegradable material (704), e.g., paste, gelatin, or Nylon 12. After reaching the targeted remote location (Fig. 47B), the biodegradable interconnecting matter dissolves, the vehicle is dissembled to its constituent enveloping portions (708, 710 in Figure 47B), allowing effecter (706), free to open and function *in situ* at the conditions of the targeted remote location.

Figure 48 shows a schematic representation of another variation of the implant, in this instance having an entire enveloping layer (712) comprising a biodegradable material.

Figure 49 shows a schematic representation of still another variation of the implant, in this instance having an inner biodegradable layer (714) enveloped by a second layer (716) comprising, e.g., a drug releasing or lubricating material.

Figure 50 shows a schematic representation of another variation of the implant, in this instance having a number or plurality of integrated enveloping sections (718). Those layers may be interconnected by bioerodible materials or other physical, mechanical, or chemical materials or components.

### BIODEGRADABLE POLYMERS

Bioerodible or biodegradable polymers include, without limitation, biocompatible polymers that are may be bioerodible by cellular action or are biodegradable by action of body fluid components, such as those found in gastric juices. Such polymeric substances include polyesters, polyamides, polypeptides, polysaccharides, and the like. Suitable biocompatible, biodegradable polymers, include polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, polymalic acid, poly(amino acids), polymethyl vinyl ether, polymaleic anhydride, chitin, chitosan, and their block and intimate copolymers, terpolymers, or higher polymer-monomer polymers and combinations and mixtures thereof. Many of the more operable biodegradable polymers are degraded by hydrolysis.

These polymers may be either surface erodible polymers such as polyanhydrides or bulk erodible polymers such as polyorthoesters. Poly(1-lactic acid) (P1LA), poly(dl-lactic acid) (PLA), poly(glycolic acid) (PGA), polycaprolactones, copolymers, terpolymer, higher poly-monomer polymers thereof, or combinations or mixtures thereof are very useful biocompatible, biodegradable polymers. One very useful biodegradable copolymer comprises a lactic acid and glycolic acid copolymers sometimes referred to as poly(dl-lactic-co-glycolic acid) (PLG). The co-monomer (lactide:glycolide) ratios of the poly(DL-lactic-co-glycolic acid) commercial materials are typically between about 100:0 to about 50:50 lactic acid to glycolic acid. Co-monomer ratios between about 85:15 and about 50:50 lactic acid to glycolic acid are quite suitable. Blends of PLA with PLG, e.g., between about 85:15 and about 50:50 PLG to PLA, are also used to prepare suitable polymer materials.

PLA, PILA, PGA, PLG, their combinations, mixtures, alloys, and blends are among the synthetic polymers approved for human clinical use. They are used as surgical suture materials and in various controlled release devices. They are biocompatible and their degradation products comprise low molecular weight compounds, such as lactic acid and glycolic acid, which enter into normal metabolic pathways. Furthermore, copolymers of poly(lactic-co-glycolic acid) offer the advantage of a large spectrum of degradation rates, the time-to-failure of a selected filament ranging from a few days to years by simply varying the copolymer ratio of lactic acid to glycolic acid.

To enhance bio-degradation of the biodegradable polymers listed above, those polymeric compositions may also include enzymes chosen to facilitate the biodegradation of those polymers. Suitable enzymes and similar reagents are proteases or hydrolases with ester-hydrolyzing capabilities. Such enzymes include proteinase K, bromelaine, pronase E, cellulase, dextranase, elastase, plasmin streptokinase, trypsin, chymotrypsin, papain, chymopapain, collagenase, subtilisn, chlostridopeptidase A, ficin, carboxypeptidase A, pectinase, pectinesterase, various oxidoreductases, various oxidases, and the like. The inclusion of an appropriate amount of such a degradation enhancing agent may be used to regulate implant erosion time.

### ENTERIC COATING MATERIAL

Enteric coating materials suitable for use in our device may be selected from known aqueous enteric film coating systems, such as aqueous dispersions of acrylic resins, especially, polymethacryl methacrylate copolymers, and dispersions of acetates, especially, cellulose acetate phthalate polymers. Suitable members include acrylic-based resins, azopolymers, polymers of polyvinylacetate and polyacrylic acid, copolymers of methacrylic acid and methylmethacrylate, methylcellulose, carboxymethylcellulose, hydroxypropylcellulose, and hydroxypropylmethylcellulose, polyvinyl acetyldiethylaminoacetate, cellulose acetatephthalate, and ethyl cellulose, and copolymers of methacrylic acid and methyl methacrylate. Adjuvants such as shellac, talc, stearic acid, and a plasticizer may also be included. Suitable compositions may be obtained by esterifying a carboxyalkyl group of carboxyalkyl cellulose, such group selected from hydroxypropylmethylcellulose (HPMC), hydroxypropylcellulose (HPC), carboxymethylcellulose, and sodium or calcium salts thereof; carraginan, anginic acid, and magnesium, sodium, and calcium salts thereof; povidone, polyvinylalcohol, tragacanth gum, chitosan, and chitin; elastic polymers, rubbers, bio-rubbers, or silicones; poly(glycerol sebacate) and/or its derivatives; fluoropolymers such as polytetrafluoroethylene (PTFE), ePTFE, and fluorinated ethylene-propylene resins (FEP); polyethylene terephthalate (PET); Hytrel polyesters; various aromatic polymers, and certain forms of polyethereketone (PEEK); various of the Nylons, especially Nylon 12; biodegradable and bioabsorbable elastomers such as hydrogels, elastin-like peptides, poly hydroxyalkanoates (PHA's), and biodegradable polymers such as poly (lactide), poly (glycolide), and their copolymers (PLGA); alginate and sodium alginate, polyethylene glycol and its derivatives.

Other suitable materials include hydrogels, acetal copolymers, and homopolymers. Acrylonitrile butadiene styrene (ABS) and mixtures of ABS with polycarbonates, polyamides, polyimides, polyacrylates, polyaryl sulfone, polycarbonates, polyetherimide, polyether sulfone, polyphenylene oxide, polyphenylene sulfide, polypropylene, polysulfone, polyurethane, polyvinyl chloride, and styrene acrylonitrile are suitable. Various materials such as animal-originated intestine, bowels or the like, starch, pasta, pre-gelatinized starch, lactose, mannitol, sorbitol, sucrose, and dextrin are suitable. Other approved materials such as Carbomer 910, 934, 934P, 940, 941, or 1342, calcium carbonate, calcium phosphate dibasic or tribasic, calcium sulfate, and talc. Amine-based polymers such as poly(allylamine hydrochloride) crosslinked with epichorohydrin arid alkylated with 1-bromodecane and (6-bromohexyl)-trimethylammonium bromide; polymers that are made by polymerizing an aliphatic amine monomer, e.g., a saturated or unsaturated, straight-chained, branched or cyclic non-aromatic hydrocarbon having an amino substituent and optionally one or more additional substituents.

### Metallic Bioerodible Materials

Metallic bioerodible materials include the metals magnesium, titanium, zirconium, niobium, tantalum, and zinc. Silicon is a bioerodible semi-metal. Mixtures and alloys of these materials are also bioerodible, e.g., certain zinc-titanium alloys, for example, as discussed in U.S. Pat. No. 6,287,332, to Bolz et al.

The physical properties of such alloys may be controlled both by selection of the metal and by selecting the relative amounts of the resulting alloy. For example, addition of about 0.1 % to 1% weight of titanium reduces the brittleness of crystalline zinc. The addition of gold to the zinc-titanium alloy at a weight percentage by weight of 0.1% to 2% reduces the alloy's grain size and raises the tensile strength of the material.

Other bioerodible metallic alloys include the materials discussed just above and one or more metals selected from lithium, sodium, potassium, calcium, iron, and manganese. The materials from the first group may form an oxidic coating that is somewhat resistive to erosion upon exposure to gastric fluids. The metals from the second group are comparatively more erodible in gastric fluids and promote the dissolution of the otherwise resistive coating.

Further details relating to the latter alloys are also found in U.S. Pat. No. 6,287,332 to Bolz et al.

Magnesium alloys are particularly suitable members of this class. The alloys, for instance, may comprise an alloy of lithium and magnesium with a magnesium-lithium ratio of about' 60:40, optionally containing fatigue-improving components such as zinc. Sodium-magnesium alloys are also suitable.

### Adjuvant compositions

The body of the implant may also comprise adjuvant compositions such as may be selected from therapeutic compositions, slow-release therapeutic compositions, medications, pH buffers, anti-acid compositions, anti-inflammatory agents, antihistamines, additives, lubricants, uv contrasting agents, ultrasound contrast agents, radio-opacifiers, diagnostic agents, digestive-related therapeutic agents, probiotic bacteria cultures or any combination thereof. Depending upon the adjuvant composition and the material in the implant body, the adjuvant may be infused into, mixed with, or coated onto the implant body.

## Claims

1. A compacted, swallowable, self-expanding, temporary gastric implant (440) comprising:
at least one expandable envelope having an interior and an exterior, configured to temporarily seal, after the implant has been swallowed, the envelope interior from gastric fluid in a human stomach at least until an integral gas-producing composition in fluid communication with the interior of the envelope produces a gas to expand the envelope to a selected size, and wherein the envelope is configured so that the selected size of the expanded envelope contacts walls of the stomach sufficiently to produce a sensation of fullness in that stomach,
wherein the integral gas-producing composition has first (448) and second (454) components and is configured to produce a gas upon being swallowed by a human being by reacting the first component with the second component,
and wherein the implant is rolled, before swallowing, to maintain the isolation of the first and second components;
**characterised in that**:
the rolled implant (460) is coated with a biodegradable material (458).

2. The gastric implant of claim 1 where the gas is carbon dioxide.

3. The gastric implant of claim 1 where the gas-producing composition comprises a base and an acid and where the gas-producing composition optionally comprises a base selected from sodium bicarbonate (NaHCO₃), sodium carbonate (Na₂CO₃), calcium bicarbonate (Ca(HCO₃)₂), calcium carbonate (CaCO₃), and their mixtures and an acid selected from vinegar, acetic acid, citrus juices, ascorbic acid, and tartaric acid, which citrus juice is optionally selected from lime, lemon, orange, and grapefruit juices, and their mixtures.

4. The gastric implant of claim 1 wherein the envelope comprises at least one bioerodible material.

5. The gastric implant of claim 4 wherein the at least one bioerodible material is selected from:
polyesters, polyamides, polypeptides, and polysaccharides; or
polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, polymalic acid, poly(amino acids), polymethyl vinyl ether, polymaleic anhydride, chitin, chitosan, and their block and intimate copolymers, terpolymers, or higher polymer-monomer polymers and combinations and mixtures thereof; or
poly(1-lactic acid) (P1LA), poly(dl-lactic acid) (PLA), poly(glycolic acid) (PGA), polycaprolactones, copolymers, terpolymers, higher poly-monomer polymers thereof, and combinations or mixtures thereof.

6. The gastric implant of claim 1 wherein the envelope is compacted by rolling or folding.

7. The gastric implant of claim 1 further comprising either:
a bioerodible capsule enclosing the envelope and gas-producing composition; or
a bioerodible coating enclosing the envelope and gas-producing composition, which bioerodible coating optionally comprises gelatin.

8. The gastric implant of claim 1 further comprising a bioerodible shape stabilizer maintaining the compacted shape of the implant.

9. The gastric implant of claim 1 further comprising a bioerodible capsule enclosing the envelope and gas-producing composition wherein the envelope, when inflated, comprises a toric shape and wherein, optionally, the expandable envelope and the gas-producing composition are rolled to a size suitable for swallowing.

10. The gastric implant of claim 1 wherein the envelope, when inflated, comprises a shape selected from: a toric shape, a cross shape, a bridged toric shape, a skeletal pyramid shape, a donut shape, a double flexed washer shape, and a Moebius strip shape.

11. The gastric implant of claim 4 further comprising an enteric coating.

12. The gastric implant of claim 1 wherein the gas-producing composition comprises an integral, frangible gas-producing composition configured to produce a gas after being swallowed by a human being and upon fracture of at least a portion of the integral, frangible gas-producing composition.

13. The gastric implant of claim 12 wherein the integral, frangible gas-producing composition comprises a base and an acid and further comprises at least one fracturable region that at least partially contains at least one of the base and the acid and said fracturable region being configured to allow contact of the base and the acid upon fracture of said fracturable region after the swallowing by a human being.

14. The gastric implant of claim 13 wherein the at least one fracturable region comprises at least one fracturable wall that at least partially contains at least one of the base and the acid, and wherein the at least one fracturable wall comprises either:
a fracturable wall that wholly contains one of the base and the acid; or
more than one fracturable wall containing the base and the acid; or
a fracturable wall that in combination with the envelope contains at least one of the base and the acid; or
more than one fracturable wall that in combination with the envelope contains the base and the acid; or
at least one fracturable joint configured to allow contact of the base and the acid upon fracture of said at least one fracturable joint after the swallowing by a human being, the at least one of the base and the acid optionally being partially contained by the envelope.

15. The gastric implant of claim 12 further comprising:
a bioerodible capsule enclosing the envelope and gas-producing composition, and
wherein the expandable envelope and the gas-producing composition are rolled to a size suitable for swallowing.

## Patentansprüche

1. Kompaktes, schluckbares, selbst ausdehnbares, temporäres Magenimplantat (440), umfassend:
mindestens eine ausdehnbare Einhüllende, welche ein Inneres und ein Äußeres aufweist, welche ausgestaltet ist, um das Innere der Einhüllenden temporär vor einem Magenfluid in einem menschlichen Magen abzudichten, nachdem das Implantat geschluckt worden ist, bis eine integrale Gas erzeugende Zusammensetzung in Fluid-Verbindung mit dem Inneren der Einhüllenden ein Gas erzeugt, um die Einhüllende auf eine bestimmte Größe auszudehnen, und
wobei die Einhüllende ausgestaltet ist, so dass die bestimmte Größe der ausgedehnten Einhüllenden Wände des Magens ausreichend berührt, um ein Empfinden einer Fülle in diesem Magen zu erzeugen,
wobei die integrale Gas erzeugende Zusammensetzung eine erste Komponente (448) und eine zweite Komponente (454) aufweist und ausgestaltet ist, um ein Gas zu erzeugen, wenn sie durch einen Menschen geschluckt wird, indem die erste Komponente mit der zweiten Komponente reagiert, und
wobei das Implantat gerollt ist, bevor es geschluckt wird, um die Isolierung der ersten und der zweiten Komponente zu erhalten;
**dadurch gekennzeichnet,**
**dass** das gerollte Implantat (460) mit einem biologisch abbaubaren Material (458) beschichtet ist.

2. Magenimplantat nach Anspruch 1, **dadurch gekennzeichnet, dass** das Gas Kohlendioxid ist.

3. Magenimplantat nach Anspruch 1, **dadurch gekennzeichnet, dass** die Gas erzeugende Zusammensetzung eine Base und eine Säure umfasst, und dass die Gas erzeugende Zusammensetzung optional umfasst eine Base, welche ausgewählt ist aus Natriumbicarbonat (NaHCO₃), Natriumcarbonat (Na₂CO₃), Calciumhydrogencarbonat (Ca(HCO₃)₂), Calciumcarbonat (CaCO₃) und ihren Gemischen, und eine Säure, welche ausgewählt ist aus Essig, Essigsäure, Zitrussäften, Askorbinsäure und Weinsäure, wobei der Zitrussaft optional ausgewählt ist aus Limonen-, Zitronen-, Orangen- und Grapefruit-Saft und ihren Gemischen.

4. Magenimplantat nach Anspruch 1, **dadurch gekennzeichnet, dass** die Einhüllende mindestens ein biologisch abbaubares Material umfasst.

5. Magenimplantat nach Anspruch 4, **dadurch gekennzeichnet, dass** das mindestens eine biologisch abbaubare Material ausgewählt ist aus:
Polyestern, Polyamiden, Polypeptiden und Polysacchariden oder Polylactiden, Polyglycoliden, Polycaprolactonen, Polyanhydriden, Polyamiden, Polyurethanen, Polyesteramiden, Polyorthoestern, Polydioxanonen, Polyacetanen, Polyketalen, Polycarbonaten, Polyorthocarbonaten, Polyphosphazenen, Polyhydroxybutyraten, Polyhydroxyvaleraten, Polyalkylenoxalaten, Polyalylensuccinaten, Poly-Äpfelsäure, Poly(aminosäuren), Polymethylvinylether, Polymaleinsäureanhydrid, Chitin, Chitosan und ihren Block- und "Intimate"-Copolymeren, Terpolymeren oder höheren Polymer-Monomer-Polymeren und Kombinationen und Gemischen davon oder Poly(I-Milchsäure)(PILA), Poly(dl-milchsäure)(PLA), Poly(glycolsäure)(PGA), Polycaprolactonen, Copolymeren, Terpolymeren, höheren Poly-Monomer-Polymeren davon und Kombinationen oder Gemischen davon.

6. Magenimplantat nach Anspruch 1, **dadurch gekennzeichnet, dass** die Einhüllende durch Rollen oder Falten kompakt angeordnet ist.

7. Magenimplantat nach Anspruch 1, darüber hinaus umfassend entweder:
eine biologisch abbaubare Kapsel, welche die Einhüllende und die Gas erzeugende Zusammensetzung einschließt;
oder
eine biologisch abbaubare Beschichtung, welche die Einhüllende und die Gas erzeugende Zusammensetzung einschließt, wobei die biologisch abbaubare Beschichtung optional Gallert umfasst.

8. Magenimplantat nach Anspruch 1, darüber hinaus einen biologisch abbaubaren Formstabilisator umfassend, welcher die kompakte Form des Implantats beibehält.

9. Magenimplantat nach Anspruch 1, darüber hinaus eine biologisch abbaubare Kapsel umfassend, welche die Einhüllende und die Gas erzeugende Zusammensetzung einschließt, wobei die Einhüllende, wenn sie aufgebläht ist, eine torische Form umfasst, und wobei die ausdehnbare Einhüllende und die Gas erzeugende Zusammensetzung optional zu einer zum Schlucken geeigneten Größe gerollt sind.

10. Magenimplantat nach Anspruch 1, **dadurch gekennzeichnet, dass** die Einhüllende, wenn sie aufgebläht ist, eine Form umfasst, welche ausgewählt ist aus: einer torischen Form, einer Kreuzform, einer überbrückten torischen Form, einer skelettartigen Pyramidenform, einer Donutform, einer doppelt gebogenen Scheibenform und einer Möbiusbandform.

11. Magenimplantat nach Anspruch 4, darüber hinaus eine magensaftresistente Beschichtung umfassend.

12. Magenimplantat nach Anspruch 1, **dadurch gekennzeichnet, dass** die Gas erzeugende Zusammensetzung eine integrale, zerbrechliche Gas erzeugende Zusammensetzung umfasst, welche ausgestaltet ist, um nach einem Schlucken durch ein menschliches Wesen und nach einem Zerbrechen von mindestens einem Teil der integralen, zerbrechlichen Gas erzeugenden Zusammensetzung ein Gas zu erzeugen.

13. Magenimplantat nach Anspruch 12, **dadurch gekennzeichnet, dass** die integrale, zerbrechliche Gas erzeugende Zusammensetzung eine Basis und eine Säure umfasst und darüber hinaus mindestens einen zerbrechlichen Bereich umfasst, welcher zumindest teilweise die Base und/oder die Säure enthält, und wobei der zerbrechliche Bereich ausgestaltet ist, um einen Kontakt der Base und der Säure beim Brechen des zerbrechlichen Bereichs nach dem Schlucken durch ein menschliches Wesen zu ermöglichen.

14. Magenimplantat nach Anspruch 13, **dadurch gekennzeichnet, dass** der mindestens eine zerbrechliche Bereich mindestens eine zerbrechliche Wand umfasst, welche zumindest teilweise die Base und/oder die Säure enthält, und dass die mindestens eine zerbrechliche Wand umfasst entweder:
eine zerbrechliche Wand, welche vollständig die Base und die Säure enthält; oder mehr als eine zerbrechliche Wand, welche die Base und die Säure enthält; oder
eine zerbrechliche Wand, welche in Kombination mit der Einhüllenden die Base und/oder die Säure enthält; oder
mehr als eine zerbrechliche Wand, welche in Kombination mit der Einhüllenden die Base und die Säure enthält; oder
mindestens eine zerbrechliche Verbindung, welche ausgestaltet ist, um einen Kontakt der Base und der Säure nach einem Brechen der mindestens einen zerbrechlichen Verbindung und nach dem Schlucken durch ein menschliches Wesen zu ermöglichen, wobei die Base und/oder die Säure optional teilweise in der Einhüllenden enthalten sind.

15. Magenimplantat nach Anspruch 12, darüber hinaus umfassend:
eine biologisch abbaubare Kapsel, welche die Einhüllende und die Gas erzeugende Zusammensetzung einschließt, und
wobei die ausdehnbare Einhüllende und die Gas erzeugende Zusammensetzung zu einer zum Schlucken geeigneten Größe gerollt sind.

## Revendications

1. Implant gastrique temporaire (440), compacté, déglutissable et auto-extensible,
comprenant au moins une enveloppe extensible présentant un intérieur et un extérieur, conçue de manière à fermer temporairement, une fois l'implant dégluti, l'intérieur de l'enveloppe de manière étanche au suc gastrique dans l'estomac d'un humain, au moins jusqu'à ce qu'une composition intégrée produisant un gaz, en communication fluidique avec l'intérieur de l'enveloppe, produise un gaz pour que l'enveloppe s'étende jusqu'à une taille sélectionnée, l'enveloppe étant conçue pour venir, lorsqu'elle s'est étendue à la taille sélectionnée, en contact avec les parois de l'estomac, à un degré suffisant pour donner une sensation de plénitude dans l'estomac,
laquelle composition intégrée produisant un gaz comprend un premier composant (448) et un deuxième composant (454) et est conçue pour produire un gaz, après déglutition par un humain, par réaction du premier composant avec le deuxième composant,
lequel implant, avant déglutition, est enroulé de manière à maintenir isolés les premier et deuxième composants,
**caractérisé en ce que**
l'implant enroulé (460) est enrobé d'un matériau biodégradable (458).

2. Implant gastrique conforme à la revendication 1, dans lequel le gaz est du dioxyde de carbone.

3. Implant gastrique conforme à la revendication 1, dans lequel la composition produisant un gaz comprend une base et un acide, et en options, la composition produisant un gaz comprend une base choisie parmi les bicarbonate de sodium (NaHCO₃), carbonate de sodium (Na₂CO₃), bicarbonate de calcium (Ca(HCO₃)₂) et carbonate de calcium (CaCO₃), ainsi que leurs mélanges, et un acide choisi parmi le vinaigre, l'acide acétique, les jus de citrus, l'acide ascorbique et l'acide tartrique, ainsi que leurs mélanges, les jus de citrus étant, en option, choisis parmi les jus de citron vert, de citron, d'orange et de pamplemousse.

4. Implant gastrique conforme à la revendication 1, dans lequel l'enveloppe comprend au moins un matériau bioérodable.

5. Implant gastrique conforme à la revendication 4, dans lequel le matériau bioérodable au nombre d'au moins un est choisi parmi :
- les polyesters, polyamides, polypeptides et polysaccharides ;
- ou les polylactides, polyglycolides, polycaprolactones, polyanhydrides, polyamides, polyuréthanes, poly(ester-amide), polyorthoesters, polydioxanones, polyacétals, polycétals, polycarbonates, polyorthocarbonates, polyphosphazènes, polyhydroxybutyrates, polyhydroxyvalérates, poly(oxalate d'alcanediyle), poly(succinate d'alcanediyle), poly(acide malique), poly(acide aminé), poly(méthyl-vinyl-éther), poly(anhydride maléique), chitine et chitosane, et les copolymères, terpolymères et polymères correspondants d'ordre plus élevé en monomères de polymère, à blocs ou statistiques, et leurs combinaisons et mélanges ;
- ou le poly(acide 1-lactique) ou PILA, le poly(acide dl-lactique) ou PLA, le poly(acide glycolique) ou PGA, les polycaprolactones, et les copolymères, terpolymères et polymères correspondants à nombre plus élevé de monomères, et leurs combinaisons et mélanges.

6. Implant gastrique conforme à la revendication 1, dans lequel l'enveloppe est compactée par enroulement ou repliement.

7. Implant gastrique conforme à la revendication 1, qui comprend en outre :
- soit une capsule bioérodable renfermant l'enveloppe et la composition produisant un gaz,
- soit un enrobage bioérodable renfermant l'enveloppe et la composition produisant un gaz, lequel enrobage bioérodable, en option, comprend de la gélatine.

8. Implant gastrique conforme à la revendication 1, qui comprend en outre un agent bioérodable de stabilisation de forme, qui maintient l'implant sous sa forme compactée.

9. Implant gastrique conforme à la revendication 1, qui comprend en outre une capsule bioérodable renfermant l'enveloppe et la composition produisant un gaz, dans lequel l'enveloppe, une fois gonflée, possède la forme d'un tore, et pour lequel, en option, l'enveloppe extensible et la composition produisant un gaz sont enroulées jusqu'à avoir une taille appropriée pour la déglutition.

10. Implant gastrique conforme à la revendication 1, dans lequel l'enveloppe, une fois gonflée, possède une forme choisie parmi les suivantes : la forme d'un tore, la forme d'une croix, la forme d'un tore à pont, la forme d'un squelette de pyramide, la forme d'un beignet, la forme d'une rondelle doublement fléchie, et la forme d'une bande de Moebius.

11. Implant gastrique conforme à la revendication 4, qui comprend en outre un enrobage entérique.

12. Implant gastrique conforme à la revendication 1, dans lequel la composition produisant un gaz comprend une composition intégrée et frangible produisant un gaz, conçue pour produire un gaz après déglutition par un humain et à la suite de la rupture d'au moins une partie de la composition intégrée et frangible produisant un gaz.

13. Implant gastrique conforme à la revendication 12, dans lequel la composition intégrée et frangible produisant un gaz comprend une base et un acide et comprend en outre au moins une région frangible qui contient au moins une partie de l'un au moins de la base et de l'acide, laquelle région frangible est conçue pour permettre à la base et à l'acide d'entrer en contact à la suite de la rupture de ladite région frangible après déglutition par un humain.

14. Implant gastrique conforme à la revendication 13, dans lequel la région frangible au nombre d'au moins une comprend au moins une paroi frangible qui contient au moins une partie de l'un au moins de la base et de l'acide, et dans lequel la paroi frangible au nombre d'au moins une comprend :
- soit une paroi frangible qui contient la totalité de l'un de la base et de l'acide,
- soit plus d'une paroi frangible, qui contiennent la base et l'acide,
- soit une paroi frangible qui, en combinaison avec l'enveloppe, contient l'un au moins de la base et de l'acide,
- soit plus d'une paroi frangible qui, en combinaison avec l'enveloppe, contiennent la base et l'acide,
- soit au moins un joint frangible conçu pour permettre à la base et à l'acide d'entrer en contact à la suite de la rupture dudit joint frangible au nombre d'au moins un après déglutition par un humain, l'un au moins de la base et de l'acide étant, en option, en partie contenu par l'enveloppe.

15. Implant gastrique conforme à la revendication 12, qui comprend en outre une capsule bioérodable renfermant l'enveloppe et la composition produisant un gaz, et pour lequel l'enveloppe extensible et la composition produisant un gaz sont enroulées jusqu'à avoir une taille appropriée pour la déglutition.
